# EUROPÄISCHE PATENTSCHRIFT

(11) **EP 3 103 410 B1**
(45) Veröffentlichungstag und Bekanntmachung des Hinweises auf die Patenterteilung: **01.05.2019**
(21) Anmeldenummer: 16173221.9
(22) Anmeldetag: 07.06.2016
(51) Int. Cl.: A61B 34/30, A61B 34/00

(54) **VORRICHTUNG UND VERFAHREN ZUR ROBOTERGESTÜTZTEN CHIRURGIE**
DEVICE AND METHOD FOR ROBOT-ASSISTED SURGERY
DISPOSITIF ET PROCÉDÉ DE CHIRURGIE ROBOTISÉ

(30) Priorität: 12.06.2015 DE 102015109371
(43) Veröffentlichungstag der Anmeldung: 14.12.2016
(73) Patentinhaber: avateramedical GmbH, 07745 Jena (DE)
(72) Erfinder: Seeber, Marcel, 07745 Jena (DE)
(74) Vertreter: Schaumburg und Partner Patentanwälte mbB

(56) Entgegenhaltungen:
- WO-A1-2006/091494
- US-A1- 2010 076 305
- US-A1- 2014 171 957

## Beschreibung

Die Erfindung betrifft eine Vorrichtung und ein Verfahren zur robotergestützten Chirurgie zur Unterstützung beim Positionieren eines Manipulatorarms in einem Koordinatensystem einer Vorrichtung zur robotergestützten Chirurgie. Die Vorrichtung hat eine Instrumenteneinheit, die ein chirurgisches Instrument mit einem Instrumentenschaft umfasst. Das proximale, dem Patienten zugewandte Ende des Instrumentenschafts ist durch eine Körperöffnung eines Patienten zu einem Zielgebiet führbar. Die Instrumenteneinheit ist mit einem Manipulatorarm der Vorrichtung verbindbar.

In der minimalinvasiven Chirurgie werden zunehmend sogenannte Telemanipulatorsysteme, die auch als Roboterassistenzsysteme oder allgemein als Vorrichtung zur robotergestützten Chirurgie bezeichnet werden, eingesetzt. Mit Hilfe einer Vorrichtung zur robotergestützten Chirurgie werden chirurgische Instrumente aufgrund von Bedieneingaben in ihrer Lage und Orientierung gesteuert. Die chirurgischen Instrumente werden ferner mechanisch, elektrisch und/oder optisch an das Telemanipulatorsystem angekoppelt, um eine aktive Positionierung und Ausrichtung des chirurgischen Instruments sowie eine gewünschte Betätigung eines chirurgischen Instruments realisieren zu können. Hierzu haben die chirurgischen Instrumente, die neben Instrumenten mit Endeffektoren auch Endoskope und zu bedienende medizinische Geräte umfassen, eine Koppelschnittstelle, die als Koppeleinheit ausgebildet sein kann und auch als Sterileinheit bezeichnet wird. Die Vorrichtung zur robotergestützten Chirurgie hat ferner mindestens einen Manipulatorarm, an dessen proximalen Ende die Koppeleinheit vorgesehen ist, mit der die Sterileinheit verbindbar ist, um die mechanische, elektrische und/oder optische Kopplung zwischen dem Manipulatorarm und dem chirurgischen Instrument zu ermöglichen.

Es sind Vorrichtungen bekannt, bei denen die Manipulatorarme und die Koppeleinheiten der Manipulatorarme nicht steril und die chirurgischen Instrumente steril sind. Das sterile Operationsfeld wird von den nicht sterilen Elementen des Telemanipulatorsystems mit Hilfe einer sterilen Abdeckung geschützt. Diese sterile Abdeckung kann eine Sterilschleuse umfassen, die zwischen der Koppeleinheit des Manipulatorarms und der Sterileinheit eines chirurgischen Instruments vorgesehen ist. Eine solche Sterilschleuse ermöglicht das sterile Abdecken der nicht sterilen Koppelelemente der Koppeleinheit des Manipulatorarms nach dem Trennen der Sterileinheit der Instrumenteneinheit von dem Manipulatorarm. Eine solche Anordnung mit einer Sterilschleuse ist beispielsweise aus DE 10 2014 117 407 A1 und DE 10 2014 117 408 A1 bekannt.

Aus dem Dokument WO 2006/091494 A1 ist eine weitere Vorrichtung zur robotergestützten Chirurgie bekannt, bei dem ein Instrument mit einem Endeffektor mit einem Manipulatorarm koppelbar ist.

Aus dem Dokument US 2010/0076305 A1 ist ein Verfahren zum Ausrichten einer Punktionsnadel bekannt. Ein Winkel zwischen einer Eintrittsstelle der Nadel und einem Tumor und der Längsachse der Nadel kann zur Prüfung der Ausrichtung der Nadel ermittelt werden. Solche Nadeln werden üblicherweise nicht mit Vorrichtungen zur robotergestützten Chirurgie eingesetzt.

Ferner ist aus dem Dokument US 7,666,191 B1 ein Telemanipulatorsystem bekannt, bei dem die nicht sterilen Manipulatorarme mittels einer Sterilfolie abgedeckt werden. Die Koppeleinheit des Manipulatorarms umfasst vier Rotationsaktuatoren, die mit einer ersten Seite eines in der Sterilfolie integrierten Steriladapters gekoppelt werden. Dieser Steriladapter umfasst vier integrierte drehbar gelagerte Übertragungsmittel, die zwischen der Koppeleinheit des Manipulatorarms und der Sterileinheit eines chirurgischen Instruments zwischengeschaltet sind.

Aus dem Dokument DE 102 42 953 A1 ist bekannt, einen Datensatz von einem Patientenkörper mit Hilfe eines bildgebenden Verfahrens zu erzeugen und in einem Koordinatensystem darzustellen. Weiterhin werden drei nicht in einer Ebene liegende Referenzpunkte mit dem Koordinatensystem in Verbindung gebracht.

Bei der Einrichtung bekannter Vorrichtungen zur robotergestützten Chirurgie werden die Eintrittspunkte der chirurgischen Instrumente bei einem auf einem Operationstisch liegenden Patienten festgelegt und davon ausgehend die mit den Manipulatorarmen gekoppelten chirurgischen Instrumente mit den Instrumentenspitzen auf die festgelegten Eintrittsstellen ausgerichtet. Das Ausrichten der Instrumente in Bezug auf ein Zieloperationsgebiet erfolgt durch das Bedienpersonal ausgehend von dessen Erfahrungsschatz. Eine technische Überwachung oder Kontrollmöglichkeit der Ausrichtung des Manipulatorarms des chirurgischen Instruments in Bezug auf das Zieloperationsgebiet ist im Stand der Technik nicht vorgesehen.

Aufgabe der vorliegenden Erfindung ist es, eine Vorrichtung und ein Verfahren zur robotergestützten Chirurgie anzugeben, bei denen eine einfache Ausrichtung der chirurgischen Instrumente einschließlich Endoskopen in Bezug auf ein vorgesehenes Zielgebiet möglich ist.

Diese Aufgabe wird durch eine Vorrichtung mit den Merkmalen des Anspruchs 1 sowie durch ein Verfahren mit den Merkmalen des unabhängigen Verfahrensanspruchs 6 gelöst. Vorteilhafte Weiterbildungen der Erfindung sind in den abhängigen Ansprüchen angegeben.

Eine erfindungsgemäße Vorrichtung zur robotergestützten Chirurgie hat eine Instrumenteneinheit, die ein chirurgisches Instrument umfasst, dessen proximales Ende durch eine Körperöffnung eines Patienten zu einem durch Koordinaten eines Koordinatensystems der Vorrichtung festgelegten Zielgebiet führbar ist.

Die Vorrichtung umfasst mindestens einen Manipulatorarm, mit dem die Instrumenteneinheit verbindbar ist. Die Vorrichtung umfasst eine Steuereinheit, die bei einer Verbindung der Instrumenteneinheit mit dem Manipulatorarm den zur Längsachse des Instrumentenschafts orthogonalen Abstandsvektor zwischen der Längsachse und dem durch die Koordinaten festgelegten Zielgebiet ermittelt und die eine erste Steuerinformation erzeugt und vorzugsweise ausgibt, wenn der Betrag des ermittelten Abstandsvektors einen ersten voreingestellten Wert hat und/oder unterschreitet. Weiterhin umfasst die erfindungsgemäße Vorrichtung eine Ausgabeeinheit, welche abhängig von der ersten Steuerinformation ein Signal ausgibt.

Durch eine solche Vorrichtung zur robotergestützten Chirurgie wird erreicht, dass der Manipulatorarm einfach derart positioniert werden kann, dass die mit dem Manipulatorarm verbundene Instrumenteneinheit in einer korrekten Position in Bezug auf eine vorgesehene Eintrittsstelle in dem Körper eines zu operierenden Patienten als auch in Bezug auf ein durch Koordinaten x_{z}, y_{z}, z_{z} in einem Koordinatensystem X, Y, Z der Vorrichtung festgelegten Zielgebiet hat. Die Position umfasst dabei insbesondere die Lage und die Ausrichtung der Längsachse eines Instrumentenschafts eines chirurgischen Instruments der Instrumenteneinheit.

Dadurch ist eine einfache Ausrichtung des Manipulatorarms der Vorrichtung zur robotergestützten Chirurgie vor einem operativen Eingriff am Patienten möglich. Hierdurch ist es möglich, dass die Positionierung und Ausrichtung der Manipulatorarme nicht durch einen Arzt sondern durch hierfür geeignetes Personal durchgeführt werden kann. Dem Arzt obliegt dann lediglich die Überprüfung der Position der Instrumenteneinheiten. Ein chirurgisches Instrument im Sinne der Erfindung sind insbesondere Endoskope, wie Stabendoskope, oder chirurgische Instrumente mit einem Endeffektor.

Vorteilhaft ist es, wenn die Steuereinheit zumindest eine zweite Steuerinformation dann erzeugt und vorzugsweise ausgibt, wenn der Betrag des ermittelten Abstandsvektors einen zweiten voreingestellten Wert hat oder unterschreitet. Durch die zweite Steuerinformation kann somit die korrekte Ausrichtung der Lage der Längsachse des Instrumentenschafts einer Instrumenteneinheit in Bezug auf das Zielgebiet angegeben werden.

Vorzugsweise ist der zweite voreingestellte Wert Null oder ein dem Wert Null angenäherter Wert, sodass die Längsachse des chirurgischen Instruments durch das Zielgebiet verläuft. Dadurch ist eine besonders einfache Positionierung des Manipulatorarms in Bezug auf seine Position im Raum und seine Orientierung in Bezug auf die Längsachse des chirurgischen Instruments möglich.

Der ermittelte Betrag des orthogonalen Abstandsvektors ist vorzugsweise der Betrag des kürzesten orthogonalen Abstandsvektors zwischen der Längsachse und dem Zielgebiet.

Weiterhin ist es vorteilhaft, wenn die Vorrichtung eine Ausgabeeinheit hat, die aufgrund der ersten Steuerinformation ein erstes akustisches und/oder ein erstes optisches Signal ausgibt und/oder die aufgrund der zweiten Steuerinformation nur ein zweites akustisches oder ein zweites optisches Signal ausgibt. Durch die optischen und/oder akustischen Signale kann eine Bedienperson einfach über die korrekte oder noch zu korrigierende Ausrichtung der mit dem Manipulatorarm verbundenen Instrumenteneinheit informiert werden, sodass auf einfache Weise die korrekte Positionierung des Manipulatorarms vor einem chirurgischen Eingriff unterstützt wird.

Ferner ist es vorteilhaft, wenn das erste akustische Signal ein an- und abschwellender Ton oder eine Tonfolge mit einer ersten Wiederholfrequenz ist und dass das zweite akustische Signal ein Dauerton ist. Die Tonfolge umfasst dabei vorzugsweise mehrere gleiche Töne. Die Wiederholfrequenz kann mit einer Verringerung des ermittelten Betrags des Abstandsvektors zunehmen, sodass eine Bedienperson über die Wiederholfrequenz über eine Annäherung bzw. Entfernung zum bzw. vom Zielgebiet akustisch informiert wird.

Die Ausgabe des akustischen Signals kann an der Steuereinheit oder einer Bedienkonsole erfolgen.

Alternativ oder zusätzlich kann das erste optische Signal ein blinkendes Lichtsignal mit einer ersten Blinkfrequenz sein und das zweite optische Signal ein Dauerlichtsignal. Dabei können das Licht des ersten Lichtsignals und das Licht des zweiten Lichtsignals dieselbe Wellenlänge bzw. dasselbe Wellenlängenspektrum haben. Das blinkende Signal kann insbesondere durch einen gepulsten Lichtstrahl erzeugt werden. Das Dauerlichtsignal kann insbesondere mit Hilfe eines kontinuierlichen Lichtstrahls erzeugt werden. Die Blinkfrequenz kann mit einer Verringerung des Betrags des Abstandsvektors zum Zielgebiet zunehmen und bei einer Vergrößerung des Betrags des Abstandsvektors zum Zielgebiet abnehmen. Die Ausgabe des optischen Signals kann an der Steuereinheit oder einer Bedienkonsole erfolgen.

Alternativ oder zusätzlich ist es vorteilhaft, wenn zum Erzeugen des ersten optischen Signals Licht mit einer ersten Wellenlänge und zum Erzeugen des zweiten optischen Signals Licht mit einer von der ersten Wellenlänge verschiedenen zweiten Wellenlänge emittiert wird. Zum Erzeugen der optischen Signale wird Licht mit einer Wellenlänge im sichtbaren Bereich eingesetzt. Vorzugsweise ist das Licht der ersten Wellenlänge rotes Licht und das Licht der zweiten Wellenlänge grünes Licht. Hierdurch erfolgt eine Information der Bedienperson über die Position und Ausrichtung des Manipulatorarms, die mit Hilfe der Lichtsignale einfach intuitiv erfasst werden kann. Die Ausgabe des optischen Signals kann an der Steuereinheit oder einer Bedienkonsole, insbesondere auf einem Bildschirm, erfolgen.

Ferner ist es vorteilhaft, wenn die Instrumenteneinheit über einen mit einer Koppeleinheit des Manipulatorarms verbundene Sterilschleuse mit dem Manipulatorarm verbindbar ist. Über diese Sterilschleuse kann eine sterile Trennung der nicht sterilen Koppeleinheit von dem sterilen Bereich erfolgen. Beim Koppeln der Sterileinheit der Instrumenteneinheit mit der Sterilschleuse werden vorzugsweise Sterilklappen der Sterilschleuse geöffnet, sodass eine direkte Verbindung zwischen Elementen der Sterileinheit und der Koppeleinheit hergestellt werden kann. Vorzugsweise hat die Sterileinheit Sterilklappen, die beim Verbinden der Sterileinheit mit der Sterilschleuse geöffnet werden, sodass eine direkte Verbindung zwischen Übertragungselementen, insbesondere von mechanischen Antriebselementen, zwischen der Koppeleinheit und der Sterileinheit möglich ist. Nach dem Trennen der Sterileinheit von der Sterilschleuse werden sowohl die Schleusenklappen als auch die Sterilklappen wieder geschlossen, sodass sowohl die Übertragungselemente der Sterileinheit steril abgedeckt sind als auch die Übertragungselemente der Koppeleinheit.

Ein zweiter Aspekt der Erfindung betrifft ein Verfahren zum Positionieren eines Manipulatorarms in einem Koordinatensystem einer Vorrichtung zur robotergestützten Chirurgie, für einen geplanten chirurgischen Eingriff, bei dem die Koordinaten x_{z}, y_{z}, z_{z} eines Zielgebiets eines Patienten ermittelt werden. Zum Positionieren eines Manipulatorarms der Vorrichtung zur robotergestützten Chirurgie insbesondere zur Vorbereitung einer Operation an einem Patienten wird eine Instrumenteneinheit mit einer Koppeleinheit des Manipulatorarms verbunden. Die Instrumenteneinheit umfasst ein chirurgisches Instrument. Die Längsachse des chirurgischen Instrumentes ist insbesondere die Längsachse des Instrumentenschafts des chirurgischen Instruments. Bei einer Verbindung der Instrumenteneinheit mit dem Manipulatorarm wird mit Hilfe der Steuereinheit der Betrag eines zur Längsachse orthogonalen Abstandsvektors zwischen der Längsachse und dem durch die Koordinaten x_{z}, y_{z}, z_{z} festgelegten Zielgebiet ermittelt. Es wird ein erstes optisches und/oder akustisches Signal ausgegeben, wenn der ermittelte Betrag einen ersten voreingestellten Wert hat und/oder unterschreitet. Dadurch wird sichergestellt, dass die Längsachse des chirurgischen Instruments sowohl durch die geplante, insbesondere bereits markierte, oder vorhandene operative Körperöffnung des Patienten als auch durch das Zielgebiet verläuft. Dadurch ist eine einfache intuitive korrekte Positionierung des Manipulatorarms insbesondere zur Vorbereitung der Vorrichtung zur robotergestützten Chirurgie für einen chirurgischen Eingriff einfach und möglich. Hierfür ist kein speziell geschultes medizinisches Fachpersonal erforderlich, insbesondere kein Arzt.

Ferner ist es vorteilhaft, wenn ein zweites optisches und/oder akustisches Signal dann ausgegeben wird, wenn der ermittelte Betrag des Abstandsvektors einen zweiten voreingestellten Wert hat oder diesen unterschreitet. Vorzugsweise ist der zweite voreingestellte Wert Null.

Der Manipulatorarm wird vorzugsweise manuell so ausgerichtet, dass die Längsachse des Instrumentenschafts durch eine geplante, insbesondere markierte, Körperöffnung des Patienten verläuft. Hierzu kann das Instrument derart ausgerichtet werden, dass die Instrumentenspitze, insbesondere ein Endeffektor, auf die Körperöffnung zeigt oder in einen in die Körperöffnung eingeführten Trokar eingeführt ist. Weiterhin wird der Manipulatorarm so ausgerichtet, dass das erste und/oder zweite optische und/oder akustische Signal ausgegeben wird.

Es ist vorteilhaft, wenn der Manipulatorarm in einem ersten Schritt so ausgerichtet wird, dass das proximale Ende des Instrumentenschafts auf eine geplante operative Körperöffnung des Patienten zeigt und somit die Längsachse des Instrumentenschafts durch die geplante oder vorhandene Körperöffnung Der Manipulatorarm wird dann in einem zweiten Schritt so bewegt, dass der mit Hilfe der Steuereinheit ermittelte Abstand zwischen der durch die geplante oder vorhandene operative Körperöffnung des Patienten verlaufenden Längsachse und dem durch die Koordinaten festgelegten Zielgebiet den zweiten voreingestellten Abstand hat oder unterschreitet, sodass das zweite optische und/oder akustische Signal ausgegeben wird. Der Manipulatorarm wird dazu automatisch durch die Vorrichtung selbst oder manuell bewegt. Auch kann ein Teil der Ausrichtbewegung durch die Vorrichtung selbst automatisch und ein Teil der Ausrichtbewegung manuell durch eine Bedienperson erfolgen.

Alternativ kann der Manipulatorarm in einem ersten Schritt so ausgerichtet werden, dass der mit Hilfe der Steuereinheit ermittelte Betrag des Abstandsvektors zwischen der Längsachse und der Koordinaten x_{z}, y_{z}, z_{z} des Zielgebiets den zweiten voreingestellten Abstand hat oder unterschreitet. Der Manipulatorarm wird dann in einem zweiten Schritt so ausgerichtet, dass die Längsachse durch die vorhandene oder geplante, insbesondere markierte, operative Körperöffnung des Patienten verläuft und dabei die Längsachse auf das Zielgebiet ausgerichtet bleibt. Dabei kann der Manipulatorarm automatisch durch die Vorrichtung selbst und/oder manuell durch eine Bedienperson im ersten und/oder zweiten Schritt bewegt werden.

Durch die aufgezeigten Lösungen der Aufgabe der Erfindung durch die unabhängigen und abhängigen Ansprüche ist es möglich, eine optimale Vorpositionierung der Instrumenteneinheit durch eine manuelle und oder automatische Vorpositionierung der Manipulatorarme der Vorrichtung zur robotergestützten Chirurgie zu erreichen.

Der Manipulatorarm kann eine Teleskopanordnung zum Bewegen der Koppeleinheit in Richtung der Teleskopachse der Teleskopanordnung haben. Die Teleskopachse verläuft parallel zur Längsachse des Instrumentenschafts, sodass sich der Instrumentenschaft entlang seiner verlängerten Längsachse beim Ausfahren und Einfahren der Teleskopanordnung bewegt.

Durch die angegebenen Lösungen erkennt der Bediener, ob die Positionierung (Instrumentenspitze zeigt auf die gewünschte Eintrittsstelle in den Situs) und die Orientierung der Längsachse des Instrumentenschafts (entsprechende Farbe des optischen Signals, beispielsweise Grün, und/oder entsprechender Ton des akustischen Signals) gleichzeitig erfolgreich eingestellt worden sind. In dieser Position kann ein in den Situs eingeführter Trokar optimal mit einer hierfür optional vorgesehenen Koppelschnittstelle des Manipulatorarms verbunden werden. In jedem Fall ist in dieser Position der Manipulatorarm in einer optimalen Ausgangslage für einen geplanten operativen Eingriff.

Vorzugsweise sind die relevanten Bereiche der Anatomie des Patienten, insbesondere das Zielgebiet, durch die Koordinaten x'_{z}, y'_{z}, z'_{z} in einem Patientenkoordinatensystem X', Y', Z' bestimmt. Der Koordinatenursprung des Patientenkoordinatensystems X', Y', Z' kann beispielsweise im Kreuzungspunkt der Medianebenen mit der dorsal liegenden Frontalebene sowie einer Transversalebene festgelegt sein. Die Koordinaten des Patientenkoordinatensystems stehen in fester bekannter Beziehung zu einem Koordinatensystem X, Y, Z der Vorrichtung zur robotergestützten Chirurgie, sodass die Koordinaten x, y, z von Elementen der Vorrichtung einfach in Koordinaten x', y', z' des Patientenkoordinatensystems X', Y', Z' umrechnen lassen und Koordinaten x', y', z' des Patientenkoordinatensystems X', Y', Z', wie z. B. die Koordinaten x'_{z}, y'_{z}, z'_{z} des Zielgebiets, sich einfach in Koordinaten x_{z}, y_{z}, z_{z} des Koordinatensystems X, Y, Z der Vorrichtung umrechnen lassen.

Beispielsweise können die Koordinaten x'_{z}, y'_{z}, z'_{z} eines Zielgebiets im Patientenkoordinatensystem X', Y', Z' dadurch bestimmt werden, dass eine manuelle Vermessung des Patienten, z. B. mit Hilfe eines Maßbandes, durchgeführt wird. Dabei liefert eine zentimetergenaue Bestimmung der Koordinaten x_{z}, y_{z}, z_{z} des Zielgebiets in den Koordinaten des Patientenkoordinatensystems X', Y', Z' und deren Transformation in Koordinaten x_{z}, y_{z}, z_{z} des Koordinatensystems X, Y, Z der Vorrichtung eine vielfache ausreichende Genauigkeit, um das chirurgische Instrument ausreichend gut positionieren und ausrichten zu können. Alternativ kann die Vermessung des Zielgebietes auch direkt in Koordinaten x_{z}, y_{z}, z_{z} des Koordinatensystems der Vorrichtung erfolgen.

Darüber hinaus liefern moderne Verfahren der Bildgebung, wie z. B. die Computertomographie oder die Magentresonanztomographie Daten, die eine genauere Bestimmung der Koordinaten im Patientenkoordinatensystem X', Y', Z' und davon ausgehend im Koordinatensystem der Vorrichtung ermöglichen.

Der Schaft der Instrumenteneinheit hat vorzugsweise eine Länge, die im ausgefahrenen Zustand der Teleskopanordnung des Manipulatorarms gerade so lang ist, dass das proximale Ende des Instrumentenschafts ca. 1 cm bis 5 cm tief in einen in den Situs eingeführten Trokar eingeführt wird. In diesen Zustand kann auch eine optional am Manipulatorarm vorhandene Trokar-Halterung mit dem Trokar verbunden werden. Im eingefahrenen Zustand der Teleskopanordnung ist das proximale Ende des Instrumentenschafts durch das Zielgebiet hindurch bewegt worden. Bei einer solchen Ausrichtung des Manipulatorarms ist die Trokar-Halterung quasi automatisch richtig zum Verbinden mit dem Trokar positioniert, wenn das proximale Ende des Instrumentenschafts in den Trokar eingeführt ist.

In Kombination mit präoperativ ermittelten Daten des Zielgebiets, insbesondere mit Hilfe einer Computertomographie oder Magentresonanztomographie, kann während eines Einricht- und Andockvorgangs mit Hilfe der Instrumenenteinheit nach der zuvor beschriebenen Vorgehensweise die Lage der Verlängerung der Längsachse des Instrumentenschafts zum Zielgebiet auf einem zusätzlichen im Blickfeld des Bedieners angebrachten Monitor dargestellt werden. Vorzugsweise wird hierzu eine zur Längsachse des Instrumentenschaftes orthogonal liegende Schnittebene durch den CT und/oder MRT-Datensatz des Patienten dargestellt. Die Schnittebene durch den CT- und/oder MRT-Datensatz geht durch das Ziel-OP-Gebiet hindurch. Damit erhält der Bediener der akustischen und optischen Information über den Abstand der Längsachse des chirurgischen Instruments zum Zielgebiet eine weitere Entscheidungshilfe, um eine optimale Einrichtung der Manipulatorarme der Vorrichtung zur robotergestützten Chirurgie für den geplanten chirurgischen Eingriff vornehmen zu können. Die Vorrichtung zur robotergestützten Chirurgie hat vorzugsweise mehrere, insbesondere vier oder fünf, Manipulatorarme, von den vorzugweise einer mit einem Endoskop verbunden wird und die weiteren Manipulatorarme mit Instrumenteneinheiten mit chirurgischen Instrumenten mit Endeffektoren zum Durchführen des chirurgischen Eingriffs. Insbesondere die für die Instrumenteneinheiten mit diesen chirurgischen Instrumenten mit Endeffektoren vorgesehenen Manipulatorarme werden vorzugsweise nacheinander mit Hilfe der erfindungsgemäßen Vorgehensweise positioniert und ausgerichtet, um nachfolgend die für den chirurgischen Eingriff erforderlichen Manipulationen vorzunehmen. Die Koordinaten des Zielgebiets können dabei vorzugsweise einen räumlichen Bereich angeben, sodass das für die Einrichtung der Manipulatorarme relevante Zielgebiet eine räumliche Dimension hat oder durch einen Punkt definiert sein. Auch kann für jedes chirurgisches Instrument ein separates Zielgebiet vorgesehen sein, wobei sich die Zielgebiete zumindest teilweise überlappen können.

Die Steuereinheit der Vorrichtung zur robotergestützten Chirurgie dient insbesondere
- zur Eingabe und Speicherung der Koordinaten des Zielgebiets im Patientenkoordinatensystem und/oder Koordinatensystem der Vorrichtung,
- zur Berechnung der Orientierung des einzusetzenden Instrumentes aus den Positionen der Segmente des Manipulatorarms
- zur Ausgabe des optischen und/oder akustischen Signals, z. B. durch Ausgabe der Zustände: Licht aus, nur grünes Licht, nur rotes Licht; oder durch die Zustände: weißes Licht ein, nur grünes Licht ein, nur rotes Licht ein; und/oder durch die Zustände: Ton aus, erste Tonfolge, zweite Tonfolge.

Besonders vorteilhaft ist es, wenn eine am Manipulatorarm vorhandene Trokar-Halterung mit einem in den Körper des Patienten eingeführten Trokar in einem ersten Schritt verbunden wird. In einem zweiten Schritt wird die Instrumenteneinheit mit der Koppeleinheit des Manipulatorarms verbunden, wobei das proximale Ende bzw. die Spitze eines Instrumentenschafts eines chirurgischen Instruments der Instrumenteneinheit in eine Öffnung des Trokars eingeführt wird. Nach dem Verbinden der Instrumenteneinheit mit der Koppeleinheit wird vorzugsweise mit Hilfe einer Steuereinheit der zur Längsachse des Instrumentenschafts des chirurgischen Instruments orthogonale Abstandsvektor zwischen dieser Längsachse und dem durch die Koordinaten festgelegten Zielgebiet ermittelt und eine erste Steuerinformation erzeugt, wenn der Betrag des ermittelten Abstandsvektors einen ersten voreingestellten Wert hat und/oder unterschreitet. Abhängig von der ersten Steuerinformation wird vorzugsweise mit Hilfe einer Ausgabeeinheit ein optisches und/oder akustisches Signal ausgegeben.

Bei einer alternativen Vorgehensweise kann in einem ersten Schritt die Instrumenteneinheit mit der Koppeleinheit des Manipulatorarms verbunden werden und in einem zweiten Schritt eine Trokar-Halterung des Manipulatorarms mit einem in den Körper des Patienten eingeführten Trokar verbunden werden, wobei beim Verbinden der Trokar-Halterung mit dem Trokar die Spitze des Instrumentenschafts des chirurgischen Instruments der Instrumenteneinheit in die Öffnung des Trokars eingeführt wird. Anschließend wird in einem dritten Schritt der Abstandsvektor wie zuvor beschrieben zwischen der Längsachse des Instrumentenschafts und dem durch die Koordinaten festgelegten Zielgebiet ermittelt und wenn der Betrag des ermittelten Abstandsvektor einen ersten voreingestellten Wert hat und/oder unterschreitet und die erste Steuerinformation erzeugt.

Bei allen zuvor beschriebenen Vorgehensweisen wird das Personal im Operationsbereich, insbesondere das Hilfspersonal, bei der optimalen Ausrichtung des chirurgischen Instruments für den jeweiligen chirurgischen Eingriff unterstützt, ohne dass hierbei das Instrument bereits über den Trokar hinaus in den Patientenkörper eingeführt wird.

Eine zusätzliche Steuerinformation kann ausgegeben werden, wenn der Betrag des ermittelten Abstandsvektors größer als der erste voreingestellte Wert ist. Abhängig von dieser zusätzlichen Steuerinformation kann ein entsprechendes zusätzliches oder alternatives Signal ausgegeben werden, sodass eine Bedienperson darüber informiert wird, dass die Längsachse relativ weit vom Zielgebiet entfernt ist.

Die Änderung der Ausrichtung und/oder Position der mit der Koppeleinheit verbundenen Instrumenteneinheit kann durch manuelle Bewegungen des Manipulatorarms derart erfolgen, dass der Abstand zwischen der Instrumentenachse und dem Zielgebiet verändert wird. Besonders ist es vorteilhaft, wenn die Frequenz eines ausgegeben akustischen und/oder optischen Signals dabei angibt, ob der Abstandsvektor größer oder kleiner wird. Bei Erreichen bzw. Unterschreiten eines voreingestellten zweiten Werts für den Betrag des Abstandsvektors wird ein zweites optisches und/oder akustisches Signal ausgegeben, das eine optimale Ausrichtung der Instrumentenachse zum Zielgebiet signalisiert.

Das Zielgebiet ist insbesondere ein Zieloperationsgebiet. Alternativ oder zusätzlich kann das Zielgebiet durch einen Zielpunkt, wie z.B. durch den Mittelpunkt eines Zieloperationsgebiets oder durch einen von der Lage eines anderen chirurgischen Instruments abhängigen Punkt, festgelegt sein. Wenn das andere chirurgische Instrument ein bereits zumindest zum Teil in den Körper des Patienten eingeführtes Endoskop, wie z.B. ein Stabendoskop, oder ein anderes bildgebendes Systems zum Erfassen von Bildern zumindest eines Ausschnitts eines Zieloperationsgebiets ist, ist es vorteilhaft, wenn das Zielgebiet von der Lage des Endoskops oder des anderen bildgebenden Systems abhängig ist. Beispielsweise kann das Zielgebiet durch einen Punkt auf der optischen Achse der optischen Elemente des Endoskops bzw. der optischen Achse der optischen Elemente des anderen bildgebenden Systems festgelegt sein. Der Zielpunkt ist insbesondere ein Punkt im Schärfentiefebereich, beispielsweise der Brennpunkt, oder ein Punkt zwischen dem Brennpunkt und dem proximalen Ende des Endoskops.

Das andere bildgebende System kann insbesondere ein auf nicht sichtbarem Licht basierendes optisches System sein, insbesondere ein Röntgensystem, ein Computertomographiesystem, ein Magnetoresonanz-Tomographiesystem oder ein anderes geeignetes bildgebendes System sein.

Als proximal wird allgemein ein dem Patienten zugewandtes Ende eines beliebigen Elements angesehen. Als distal wird allgemein ein dem Patienten abgewandtes Ende eines beliebigen Elements angesehen.

Weitere Merkmale und Vorteile ergeben sich aus der folgenden Beschreibung, die die Erfindung anhand von Ausführungsbeispielen im Zusammenhang mit den beigefügten Figuren näher erläutert.

Es zeigen:
- Figur 1: eine schematische Seitenansicht eines Systems zur robotergestützten Chirurgie mit einem Manipulator, der vier Manipulatorarme hat, mit denen jeweils eine Instrumenteneinheit verbindbar ist;
- Figur 2: eine schematische Vorderansicht des Systems nach Figur 1;
- Figur 3: eine Anordnung zum Verbinden einer in einem sterilen Bereich einer angeordneten Instrumenteneinheit mit einer nicht sterilen Koppeleinheit eines Manipulatorarms;
- Figur 4: einen Abschnitt des Manipulatorarms mit der Koppeleinheit und der mit der Koppeleinheit verbundenen Instrumenteneinheit mit einer ausgefahrenen Teleskopanordnung des Manipulatorarms gemäß einer ersten Ausführungsform;
- Figur 5: die Anordnung nach Figur 4, wobei die Teleskopanordnung eingefahren ist, sodass das chirurgische Instrument bis in das Zieloperationsgebiet geführt ist oder über dieses hinausgeht;
- Figur 6: die schematische Darstellung der Instrumenteneinheit und des Zieloperationsgebietes in einem Koordinatensystem der Vorrichtung;
- Figur 7: eine schematische Darstellung zur Ausrichtung der mit dem Manipulatorarm verbundenen Instrumenteneinheit zum Zieloperationsgebiet nach einer ersten Vorgehensweise;
- Figur 8: eine schematische Darstellung zur Ausrichtung der mit dem Manipulatorarm verbundenen Instrumenteneinheit zum Zieloperationsgebiet nach einer zweiten Vorgehensweise;
- Figur 9: einen Ausschnitt eines Patientenkörpers mit vier markierten Positionen für geplante Körperöffnungen des Patienten, an denen jeweils ein Trokar eingeführt wird;
- Figur 10: eine Anordnung mit einem Abschnitt eines Manipulatorarms mit einer Koppeleinheit und der mit der Koppeleinheit verbundenen Instrumenteneinheit mit eingefahrener Teleskopanordnung des Manipulatorarms gemäß einer zweiten Ausführungsform mit einer Trokar-Halterung;
- Figur 11: die Anordnung nach Figur 10 mit ausgefahrener Teleskopanordnung, wobei die Trokar-Halterung des Manipulatorarms mit einem in einem Patienten eingeführten Trokar verbunden ist;
- Figur 12: die Anordnung nach Figur 11 mit eingefahrener Teleskopanordnung;
- Figur 13: eine Anordnung mit einem Abschnitt eines Manipulatorarms mit einer Koppeleinheit und einer mit der Koppeleinheit verbundenen Instrumenteneinheit mit ausgefahrener Teleskopanordnung des Manipulatorarms gemäß einer dritten Ausführungsform mit einer Trokar-Halterung zur Verbindung des Manipulatorarms mit einem in den Körper des Patienten eingeführten Trokar und mit einem über einen weiteren Trokar in den Körper des Patienten zumindest teilweise eingeführten Endoskop;
- Figur 14: die Anordnung nach Figur 13, wobei die Lage der ausgefahrenen Teleskopanordnung mit Hilfe eines Koppelgetriebes verändert worden ist, so dass die Längsachse eines chirurgischen Instruments der Instrumenteneinheit zu einem festgelegten Zielgebiet im Körper des Patienten verläuft; und
- Figur 15: die Anordnung nach Figur 13 mit eingefahrenen Teleskopanordnung.

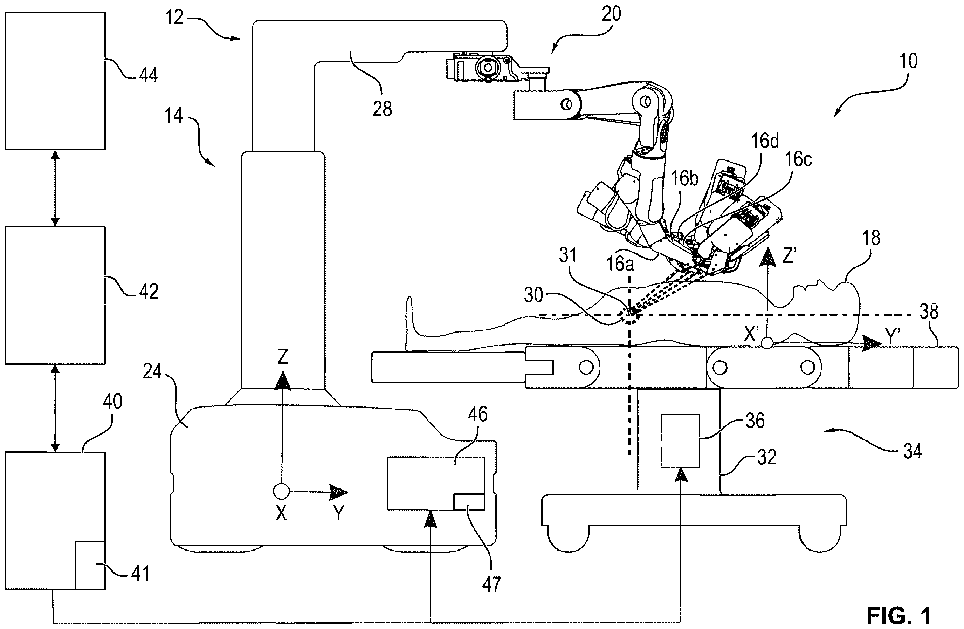

Figur 1 zeigt eine schematische Seitenansicht eines Systems 10 zur robotergestützten Chirurgie mit einem Manipulator 12, der ein Stativ 14 und vier Manipulatorarme 16a bis 16d hat. Der Manipulator 12 wird allgemein auch als Vorrichtung zur robotergestützten Chirurgie bezeichnet. Das System 10 dient zur Durchführung eines chirurgischen Eingriffs an einem auf einem Operationstisch 34 positionierten Patienten 18. Ausgehend von der Anatomie des Patienten 18 und der durchzuführenden Operation sind die Koordinaten x'_{z}, y'_{z}, z'_{z} eines Zieloperationsgebiets 30 in einem Patientenkoordinatensystem X', Y', Z' ermittelt und diese Koordinaten x'_{z}, y'_{z}, z'_{z} voreingestellt gespeichert worden. Der Manipulator 12 hat ein Koordinatensystem X, Y, Z der Vorrichtung, dessen Koordinatenursprung in einem Stativfuß 24 eines Stativs 14 des Manipulators 12 angeordnet ist. Das Stativ 14 hat einen L-förmigen Stativarm 28, an dessen vom Stativfuß 24 entfernten Ende die Manipulatorarme 16a bis 16d über einen Stativkopf 20 verbunden sind.

Der Operationstisch 34 hat eine Operationstischsäule 32, in der eine Steuereinheit 36 des Operationstischs 34 angeordnet ist und auf der eine mehrere Segmente umfassende Patientenlagerfläche 38 angeordnet ist. Die Steuereinheit 36 dient zur Steuerung der Bewegung von Elementen des Operationstischs 34, insbesondere zur Längenverstellung der Operationstischsäule 32 und somit zum Verstellen der Höhe der Patientenlagerfläche 38 und zum Verstellen einzelner Segmente sowie der Neigung und der Kantung der Patientenlagerfläche 38. Vorzugsweise ist jedoch die Verstellung der Segmente des Operationstischs 34 während eines operativen Eingriffs mit Hilfe des Manipulators 12 gesperrt. Das System 10 umfasst ferner eine Steuereinheit 46 des Manipulators 12 sowie eine zentrale Steuereinheit 40, die über Datenleitungen mit der Steuereinheit 46 des Manipulators 12, der Steuereinheit 36 des Operationstischs 34 sowie einer Bedienkonsole 42 mit einer Anzeigeeinheit 44 verbunden ist. Die Steuereinheit 40 hat eine Ausgabeeinheit 41 und die Steuereinheit 46 hat eine Ausgabeeinheit 47, durch die jeweils optische und/oder akustische Signale ausgegeben werden können.

Die Oberfläche der Patientenlagerfläche 38 bildet eine Frontalebene, auf der der Patient 18 dorsal liegend positioniert ist. Ferner verläuft durch den Koordinatenursprung des Patientenkoordinatensystems X', Y', Z' eine Transversalebene, in der die Koordinatenachsen X' und Z' liegen. Ferner verläuft durch den Koordinatenursprung eine Medianebene, in der die Koordinatenachsen Z' und Y' liegen.

Die Koordinaten x'_{z}, y'_{z}, z'_{z} des Zieloperationsgebiets 30 in dem Patientenkoordinatensystem X', Y', Z' sind bekannt und können durch die bekannte Lage des Patientenkoordinatensystems X', Y', Z' zum Koordinatensystem X, Y, Z der Vorrichtung 12 einfach bei der Ansteuerung der Manipulatorarme 16a bis 16d sowie der mit den Manipulatorarmen 16a bis 16d verbundenen Instrumenteneinheit zur Durchführung eines chirurgischen Eingriffs mit Hilfe des Manipulators 12 berücksichtigt werden, insbesondere in Koordinaten x_{z}, y_{z}, z_{z} des Koordinatensystems X, Y, Z der Vorrichtung 12 umgerechnet werden.

Die Position der Koordinaten y'_{z}, z'_{z} der Mitte des Zieloperationsgebiets 30 sind in Figur 1 in Bezug auf die Koordinatenachsen Y' und Z' mit Hilfe der durch das Zielkoordinatengebiet 30 verlaufenden Strichlinien angedeutet.

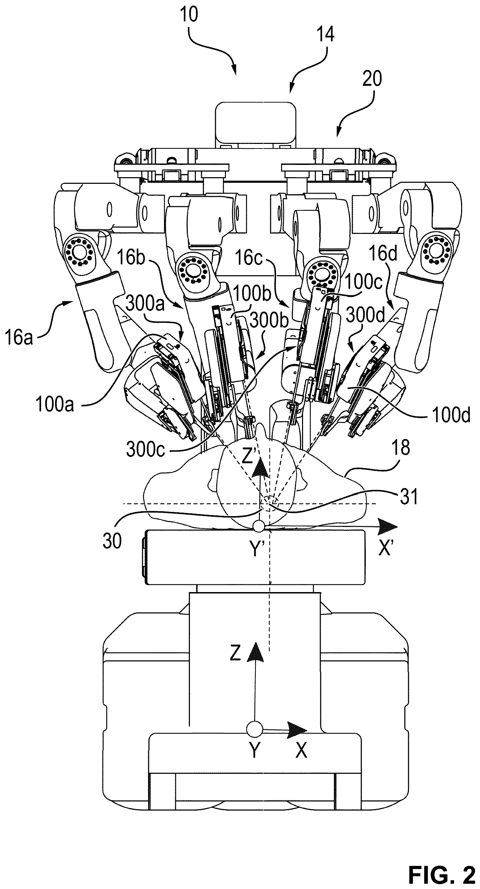

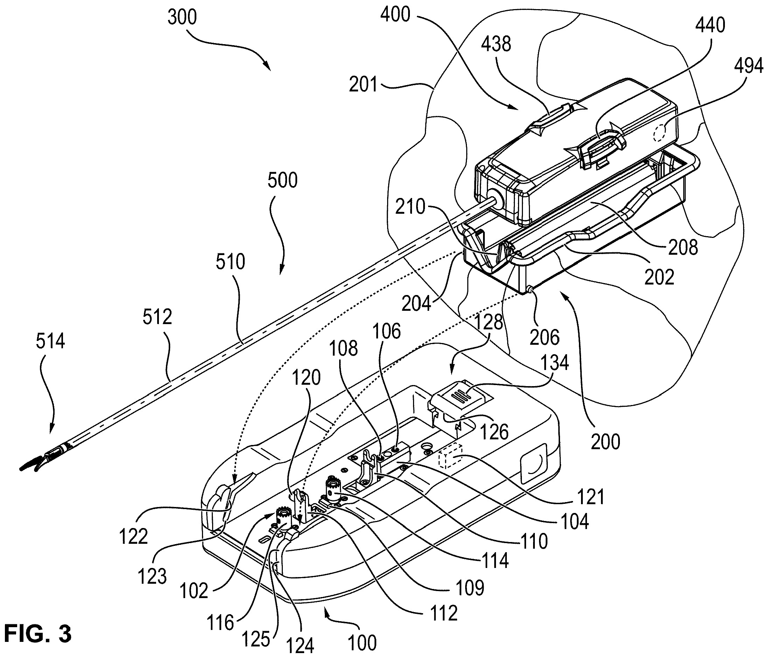

Figur 2 zeigt eine schematische Vorderansicht des Systems 10 nach Figur 1. Am proximalen Ende der Manipulatorarme 16a bis16d ist jeweils eine Koppeleinheit 100a bis 100d angeordnet, mit den jeweils eine Instrumenteneinheit 300a bis 300d zum Durchführen des chirurgischen Eingriffs verbunden ist. Die Instrumentenschäfte des jeweiligen chirurgischen Instruments der Instrumenteneinheiten 300a bis 300d sind durch Strichlinien angedeutet, die in Figur 2 von den Koppeleinheiten 100a, 100b, 100c, 100d und den mit den Koppeleinheiten 100a, 100b, 100c, 100d verbundenen in Figur 3 dargestellten Sterileinheiten 400a, 400b, 400c, 400d der Instrumenteneinheiten 300a, 300b, 300c, 300d bis ins Zieloperationsgebiet 30 reichen. Die Strichlinien geben dabei die Längsachsen bzw. verlängerten Längsachsen der Instrumentenschäfte an. Mit Hilfe der durch das Zieloperationsgebiet 30 verlaufenden und parallel zu den Koordinatenachsen X' und Z' verlaufenden Strichlinien sind die Koordinaten y'_{z}, z'_{z} des Mittelpunkts 31 des Zieloperationsgebiets 30 in Bezug auf die Koordinatenachsen X' und Z' angedeutet.

Im Folgenden wird unter Bezugnahme auf den Manipulatorarm 16a die Kopplung der Instrumenteneinheit 300a über eine Sterilschleuse 200a mit der Koppeleinheit 100a des Manipulatorarms 16a beschrieben. Die Ausführungen gelten in gleicher Weise für die weiteren Manipulatorarme 16b bis 16d und für die mit diesen Manipulatorarmen 16b bis 16d über Sterilschleusen 200b bis 200d verbundene Instrumenteneinheiten 300b bis 300d. Zur Vereinfachung werden nachfolgend die Bezugszeichenziffern ohne die zur Unterscheidung zwischen den einzelnen Manipulatorarmen verwendeten Kleinbuchstaben zu benutzen.

Figur 3 zeigt die Koppeleinheit 100 des Manipulatorarms 16, die Sterilschleuse 200 sowie die Instrumenteneinheit 300 mit der Sterileinheit 400 und einem chirurgischen Instrument 500, das einen Endeffektor 514 hat. Die Koppeleinheit 100, die Sterilschleuse 200 sowie die Instrumenteneinheit 300 sind vor dem Zusammenfügen der Sterilschleuse 200 mit der Koppeleinheit 100 und vor dem nachfolgenden Zusammenfügen der Sterileinheit 400 mit der Sterilschleuse 200 gezeigt. Eine als Sterilfolie 201 ausgeführte flexible sterile Hülle ist an einem umlaufenden Anschlussrand 202 der Sterilschleuse 200 mit dieser über eine geeignete Verbindung, wie eine Klemm-, Klebe-, Klett- und/oder Schweißverbindung fest verbunden, sodass die Sterilfolie 201 zusammen mit der Sterilschleuse 200 eine geschlossene sterile Abdeckung um die in einen sterilen Operationsbereich ragenden Bereiche des Manipulatorarms 16 bildet.

Zur besseren Darstellung ist in Figur 3 nur ein Ausschnitt der Sterilfolie 201 um die Sterilschleuse 200 herum dargestellt. In den nachfolgenden Figuren ist die Sterilfolie 201 teilweise nicht dargestellt.

Zur Kopplung der Sterileinheit 400 mit der Koppeleinheit 100 ist die Sterilschleuse 200 zwischen der Sterileinheit 400 und der Koppeleinheit 100 angeordnet und ermöglicht im gekoppelten Zustand der Sterileinheit 400 mit der Koppeleinheit 100 eine direkte Kopplung eines ersten Übertragungsmittels 102 der Koppeleinheit 100 und eines zweiten nicht dargestellten Übertragungsmittels der Sterileinheit 400.

Mit Hilfe des ersten Übertragungsmittels 102 wird im vorliegenden Ausführungsbeispiel sowohl mechanische Energie als auch elektrische Energie zwischen der Koppeleinheit 100 und der Sterileinheit 400 übertragen. Hierzu hat das erste Übertragungsmittel 102 der Koppeleinheit 100 beispielsweise mindestens vier mechanische Antriebselemente 110 bis 116 und das zweite Übertragungsmittel der Sterileinheit 400 vier zu den Antriebselementen 110 bis 116 komplementäre angetriebene Elemente. Weiterhin hat das erste Übertragungsmittel 102 ein elektrisches Übertragungselement 104 mit zwei elektrischen Kontakten 106, 108 und das zweite Übertragungsmittel ein zum elektrischen Übertragungselement 104 des ersten Übertragungsmittels 102 komplementäres elektrisches Übertragungselement.

Das erste Übertragungselement 102 umfasst ferner ein optisches Übertragungsmittel 109 zur Übertragung von Licht und/oder optischen Signalen. Die Antriebselemente des ersten Übertragungsmittels 102 umfassen ein erstes translatorisches Antriebselement 110 und ein zweites translatorisches Antriebselement 112 jeweils zur Übertragung einer translatorischen Bewegung sowie ein erstes rotatorisches Antriebselement 114 und ein zweites rotatorisches Antriebselement 116 zur Übertragung einer Rotationsbewegung. Das erste und das zweite translatorische Antriebselement 110, 112 sind jeweils als Linearhubgabel und das erste und das zweite rotatorische Antriebselement 114, 116 sind als Antriebsritzel mit stirnseitiger Zahnung ausgebildet. Ferner hat die Koppeleinheit 100 einen in einer Vertiefung angeordneten Koppelsensor, der ein durch einen aus der Sterileinheit 400 vorstehenden ersten Detektionsstift gebildetes erstes Detektionselement detektiert, wenn die Sterilschleuse 200 korrekt mit der Koppeleinheit 100 und die Sterileinheit 400 korrekt mit der Sterilschleuse 200 gekoppelt ist.

Bei anderen Ausführungsbeispielen können die ersten und zweiten Übertragungsmittel auch mehr oder weniger Antriebselemente, angetriebe Elemente und elektrische Übertragungselemente umfassen, die mechanische und/oder elektrische Energie durch direkte Kopplung übertragen. Als direkte Kopplung wird dabei eine Kopplung der Übertragungsmittel angesehen, bei der keine weiteren Übertragungselemente zwischen den ersten Übertragungsmitteln und den zweiten Übertragungsmitteln für eine Übertragung von mechanischer und/oder elektrischer Energie und/oder optischen Strahlen vorgesehen sind, wobei insbesondere keine elektrischen, mechanischen oder optischen Übertragungselemente in einer zwischen der Koppeleinheit 100 und der Sterileinheit 400 angeordneten Sterilbarriere, wie der Sterilschleuse 200, vorgesehen sind. Die Koppeleinheit 100 hat ferner eine RFID-Lese-und-Schreibeinheit 121, mit deren Hilfe ein RFID-Transponder 494 der Sterileinheit 400 lesbar und/oder schreibbar ist.

Zum Verbinden der Koppeleinheit 100 mit der Sterilschleuse 200 hat die Koppeleinheit 100 einander gegenüberliegende Führungsnuten 122, 124, in die Führungsstifte 204, 206 der Sterilschleuse 200 eingeführt werden, bis sie das vordere Ende 123, 125 der jeweiligen Führungsnut 122, 124 erreicht haben. Die Führungsstifte 204, 206 ragen an einem ersten Ende der Sterilschleuse 200 auf gegenüberliegenden Seiten nach außen. Anschließend wird das gegenüberliegende zweite Ende der Sterilschleuse 200 nach unten gedrückt, so dass die Sterilschleuse 200 um eine durch die Führungsstifte 204, 206 verlaufende Drehachse gedreht wird, bis eine Rastnase 126 eines Rastelements 128 in einen komplementären Rastbereich der Sterilschleuse 200 eingreift.

Die Entriegelungstaste 134 ist um eine Drehachse schwenkbar angeordnet und wird durch eine Feder in ihrer in Figur 3 gezeigten Rastposition gehalten. Zum Lösen der Rastverbindung wird mit einem Finger auf eine Entriegelungstaste 134 des Rastelements 128 gedrückt, so dass eine Feder gespannt und das Rastelement 128 zusammen mit der Rastnase 126 verschwenkt wird, so dass die Rastnase 126 außer Eingriff mit dem komplementären Rastelement der Sterilschleuse 200 gebracht wird. Dadurch kann das zuvor mit der Rastnase 126 in Eingriff stehende zweite Ende der Sterilschleuse 200 aus der Koppeleinheit 100 wieder herausgeschwenkt werden. Nachdem dieses zweite Ende der Sterilschleuse 200 aus der Koppeleinheit 100 herausgeschwenkt worden ist, kann die Sterilschleuse 200 wieder vollständig von der Koppeleinheit 100 getrennt werden, indem die Sterilschleuse 200 mit den mit den Führungsnuten 122, 124 in Eingriff stehenden Führungsstiften 204, 206 entlang der Führungsnuten 122, 124 aus diesem heraus gezogen wird, bis die Führungselemente 204, 206 nicht mehr in Eingriff mit den Führungsnuten 122, 124 stehen. Zwischen den Führungsnuten 122, 124 und dem Rastelement 128 ist ein durch eine entsprechende Vertiefung im Gehäuse der Koppeleinheit 100 gebildeter Aufnahmebereich vorhanden, der im vorliegenden Ausführungsbeispiel die Sterilschleuse 200 auf drei Seiten und bodenseitig zumindest teilweise umgibt.

Die Sterilschleuse 200 hat Schleusenklappen 208, 210, die über Scharniere schwenkbar sind. Mit Hilfe dieser Scharniere sind die Schleusenklappen 208, 210 von dem in Figur 3 gezeigten geschlossenen Zustand in einen geöffneten Zustand schwenkbar. Im geöffneten Zustand der Schleusenklappen 208, 210 kann eine direkte Kopplung des ersten Übertragungsmittels 102 der Koppeleinheit 100 mit dem zweiten Übertragungsmittel der Sterileinheit 400 erfolgen.

An den Außenseiten der Seitenwände und der Stirnwände der Sterilschleuse 200 ist ein umlaufender Rand 202 ausgebildet, mit dem die Sterilfolie 201 der sterilen Abdeckung auf geeignete Art und Weise verbunden ist.

Die Sterileinheit 400 hat ferner gegenüberliegend angeordnete Rast- und Betätigungselemente 438, 440 durch die automatisch eine wieder lösbare Rastverbindung beim Verbinden der Sterileinheit 400 mit der Sterilschleuse 200 erfolgt.

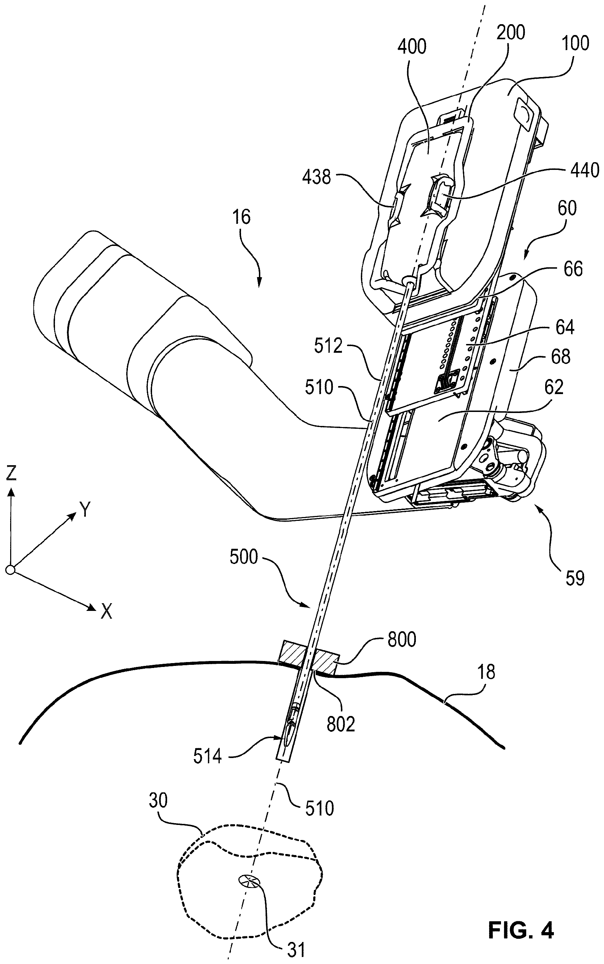

Figur 4 zeigt einen Abschnitt des Manipulatorarms 16 an dessen proximalen Ende die Koppeleinheit 100 über eine Teleskopanordnung 60 verbunden ist. Die Teleskopanordnung 60 hat zueinander verschiebbare Abschnitte 62, 64, 66 und ist in Figur 4 in einem ausgefahrenen Zustand dargestellt. Die Abschnitte 62, 64, 66 der Teleskopanordnung 60 können mit Hilfe einer Antriebseinheit 68 eingefahren und ausgefahren werden, sodass der Endeffektor 514 des chirurgischen Instruments 500 entlang der Längsachse 510 des Instrumentenschafts 512 bewegt werden kann. Der Manipulatorarm 16 hat mehrere zueinander bewegbare Segmente, deren Position relativ zueinander geändert werden kann. Die Teleskopanordnung 60 ist ferner über ein Koppelgetriebe 59 mit den weiteren Segmenten des Manipulatorarms 16 schwenkbar verbunden, sodass die Lage und Ausrichtung der Längsachse 510 des Instrumentenschafts 512 des chirurgischen Instruments 500 in seiner Position, d. h. sowohl in seiner Ausrichtung als auch in seiner Lage, durch eine Bedieneingabe an der Bedienkonsole 42 geändert werden kann. Zum Einrichten jedes Manipulatorarms 16 vor einer Operation ist die Koppeleinheit 100 derart auszurichten, dass die Längsachse 510 des Instrumentenschafts 512 des mit der Koppeleinheit 100 verbundenen chirurgischen Instruments 500 durch eine geplante oder vorhandene Körperöffnung 802 des zu operierenden Patienten 18 hindurch und durch ein festgelegtes Zieloperationsgebiet 30 verläuft. In Figur 4 ist an der Körpereintrittsstelle 802 ein Trokar 800 in den Körper des Patienten 18 eingeführt, durch den dann zum Durchführen des chirurgischen Eingriffs der vordere Teil des Schafts 512 des chirurgischen Instruments 500 zusammen mit dem Endeffektor 514 bis zum Zieloperationsgebiet 30 geführt wird.

Vor einer Operation wird der Manipulatorarm 16 zusammen mit der mit der Koppeleinheit 100 des Manipulatorarms 16 automatisch oder durch eine Bedienperson so ausgerichtet, dass dessen Längsachse 510 des Instrumentenschafts 512 durch die Öffnung des Trokars 800 verläuft. Dabei kann der Endeffektor 514 des chirurgischen Instruments 500 in die Öffnung des Trokars 800 einführt werden, sodass eine mittige Ausrichtung der Längsachse 510 auf die gewünschte oder vorhandene Körpereintrittsstelle 802 erfolgt. Ferner ermittelt die Steuereinheit 46 des Manipulators und/oder die zentrale Steuereinheit 40 den Betrag des Abstandsvektors zwischen der Längsachse 510 des Instrumentenschafts 514 zum Zieloperationsgebiet 30, insbesondere den Betrag des orthogonalen Abstandsvektors von der Längsachse 510 zum Zieloperationsgebiet 30. Dabei ist es möglich, sowohl den Betrag des Abstandsvektors zum Rand des Zieloperationsgebiets 30 und alternativ oder zusätzlich den Betrag des Abstandsvektors zum Mittelpunkt 31 des Zieloperationszielgebiets 30 zu bestimmen.

Im vorliegenden Ausführungsbeispiel verläuft die Längsachse 510 durch den Mittelpunkt 31 des Zieloperationsgebiets 30, sodass der Betrag des Abstandsvektors im vorliegenden Ausführungsbeispiel zwischen Längsachse 510 und dem zum Zieloperationsgebiet 30 Null beträgt, da die Längsachse 510 durch das Zieloperationsgebiet 30 verläuft. Auch zum Mittelpunkt 31 des Zieloperationsgebiets 30 beträgt der Abstand Null, da die Längsachse 510 durch den Mittelpunkt 31 des Zieloperationsgebiets 30 verläuft. Wenn der Betrag des orthogonalen Abstandsvektors einen ersten Wert unterschreitet, kann einer Bedienperson ein erstes optisches und/oder akustisches Signal ausgegeben werden. Sobald der Betrag des Abstandsvektors einen zweiten Wert erreicht oder unterscheidet, kann ein zweites optisches und/oder akustisches Signal ausgegeben werden. Der zweite Wert kann insbesondere Null sein, sodass das zweite optische und/oder akustische Signal dann ausgegeben wird, wenn die Längsachse 510 durch das Zieloperationsgebiet 30 oder dessen Mittelpunkt 31 verläuft. Auch kann sich das ausgegebene optische und/oder akustische Signal abhängig vom ermittelten Betrag des Abstandsvektors kontinuierlich mit Änderungen des Abstands oder in mehreren Schritten ändern, sodass der Bedienperson akustisch und/oder optisch informiert wird, ob sich die Längsachse 510 vom Zieloperationsgebiet 30 entfernt oder sich diesem annähert. Zu beachten ist, dass während der Ausrichtung des Manipulatorarms 16 bzw. bei dem Einrichtungsvorgang der Vorrichtung zur robotergestützten Chirurgie nur die Längsachse 510 des Instrumentenschafts 512 nicht der Instrumentenschaft 512 selbst durch das Zieloperationsgebiet 30 oder in einem seitlichen Abstand am Zieloperationsgebiet 30 vorbeigeführt wird.

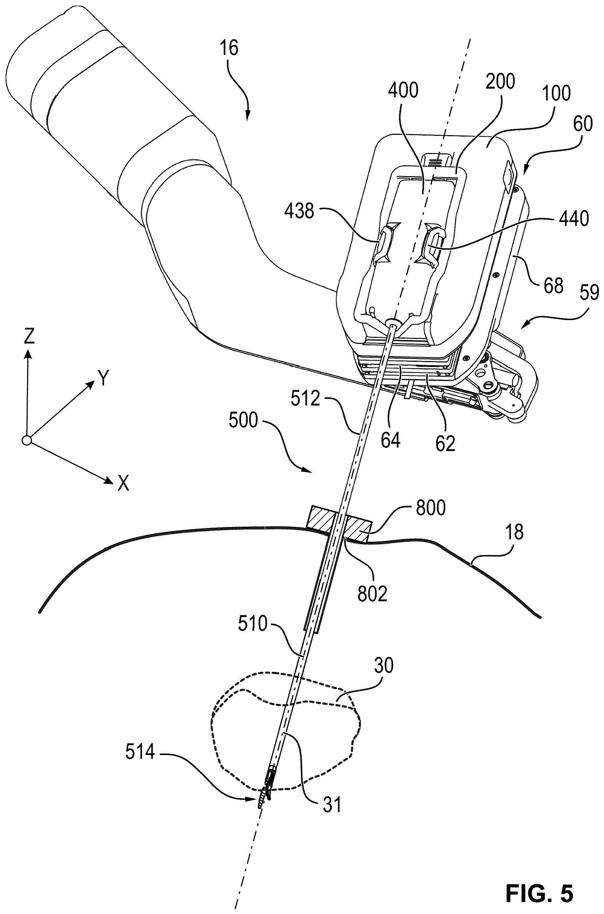

Figur 5 zeigt die Anordnung nach Figur 4, wobei die Abschnitte 62 bis 66 der Teleskopanordnung 60 im Unterschied zu Figur 4 in einem eingefahrenen Zustand dargestellt sind. Der Manipulatorarm 16 wird beim Einrichten vorzugsweise so positioniert, dass der Endeffektor 514 des Instrumentenschafts 512 des chirurgischen Instruments 500 in den in den Körper des Patienten 18 eingeführten Trokars 800 ragt, wenn die Teleskopanordnung 60 eingefahren ist. Dabei wird der Manipulatorarm 16 so positioniert, dass der Endeffektor 514 vorzugsweise mit einer Länge im Bereich von 1 cm bis 5 cm, vorzugsweise in einem Bereich zwischen 1,5 cm und 3 cm, insbesondere 2 cm, in den Trokar 800 ragt.

Von der in Figur 4 gezeigten Position aus kann die Teleskopanordnung 60 eingefahren werden, sodass der Endeffektor 514 des chirurgischen Instruments 500 durch den Trokar 800 bis in das Zieloperationsgebiet 30 geführt wird. Wie in Figur 5 gezeigt ist, kann der Endeffektor 514 auch durch das Zieloperationsgebiet 30 in Richtung der Längsachse 510 des Instrumentenschafts 512 hindurch und über das Zieloperationsgebiet 30 hinaus bewegt werden.

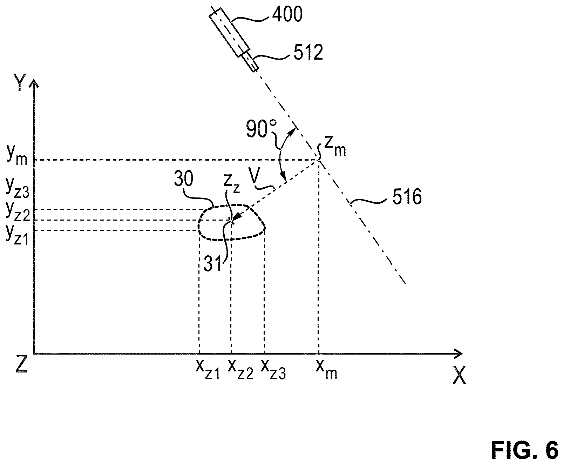

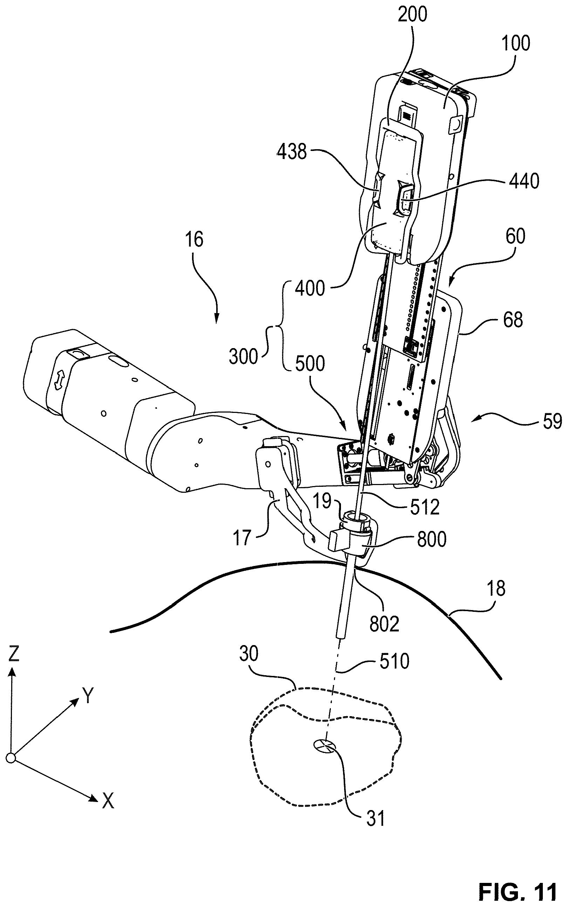

Figur 6 zeigt die schematische Darstellung der Sterileinheit 400 der Instrumenteneinheit 300 zusammen mit dem Instrumentenschaft 512 des chirurgischen Instruments 500 und des Zieloperationsgebiets 30 in dem Koordinatensystem X, Y, Z der Vorrichtung bzw. des Manipulators 12. Die räumliche Ausdehnung des Zieloperationsgebiets 30 wurde mit Hilfe eines geeigneten bildgebenden Verfahrens für den konkreten Patienten 18 ermittelt und kann dabei als einfache geometrische Körper, wie durch eine Kugel, oder durch die konkrete räumliche Ausdehnung eines für einen speziellen chirurgischen Eingriff an dem Patienten 18 ermitteltes und/oder festgelegtes Zieloperationsgebiet 30 durch eine Vielzahl von Koordinaten festgelegt sein. Das Zieloperationsgebiet 30 kann für einen Patienten 18 insbesondere mit Hilfe eines bildgebenden Verfahrens ermittelt bzw. bei Auswertung der ermittelten Bilder automatisch oder von einer Bedienperson festgelegt werden. Als bildgebendes Verfahren kann ein Röntgenverfahren, ein Computertomographieverfahren, ein Magnetresonanzverfahren oder ein anderes geeignetes Verfahren eingesetzt werden. Die Außenabmessungen des Zieloperationsgebiets 30 sind in den in Figur 11 gezeigten zweidimensionalen Koordinatensystem 26 durch die Koordinaten x_{Z1} und x_{Z3} in X-Richtung sowie durch die Koordinaten y_{Z1} und y_{Z3} auf der X-Achse festgelegt. Der Mittelpunkt 31 des ermittelten Zieloperationsgebiets 30 ist durch die Koordinate x_{Z2} auf der X-Achse und y_{Z2} auf der Y-Achse festgelegt. In gleicher Weise ist die räumliche Ausdehndung des Zieloperationsgebiets 30 auf der orthogonal zur Bildebene verlaufenden Z-Achse bekannt bzw. festgelegt. Die Koordinaten des Schnittpunkts des Vektors V mit der Längsachse 510 des Instrumentenschafts 512 sind in Figur 6 mit xₘ, yₘ, zₘ bezeichnet.

Wie bereits erläutert, ist der Manipulatorarm 16 zusammen mit der Koppeleinheit 100 vor einem chirurgischen Eingriff am Patienten 18 so zu positionieren, dass die Längsachse 510 des Instrumentenschafts 512 durch eine gewünschte Körperöffnung 802 trifft und die Längsachse 614 durch das Zieloperationsgebiet 30 verläuft. Um insbesondere eine Bedienperson bei der korrekten Ausrichtung des Manipulatorarms 16 und der Koppeleinheit 100 zu unterstützen, ermittelt die Steuereinheit 46 den Betrag des zur Längsachse 510 kürzesten dreidimensionalen orthogonalen Abstandsvektors V zwischen der Längsachse 510 und dem Mittelpunkt 31 des Zieloperationsgebiets 30. Erreicht oder unterschreitet der Betrag des Abstandsvektors V einen ersten Wert, wird ein erstes optisches und/oder akustisches Signal ausgegeben, erreicht oder unterschreitet er einen zweiten Wert, so wird ein zweites optisches und/oder akustisches Signal ausgegeben, sodass der Bedienperson eine optische und/oder akustische Information über die korrekte Ausrichtung der Koppeleinheit 100 gegeben wird. Hierdurch ist eine einfache und komfortable Möglichkeit geschaffen, eine Bedienperson bei der Einrichtung des Manipulators 12 und bei der Positionierung der Manipulatorarme 16a bis 16d vor dem eigentlichen chirurgischen Eingriff zu unterstützen.

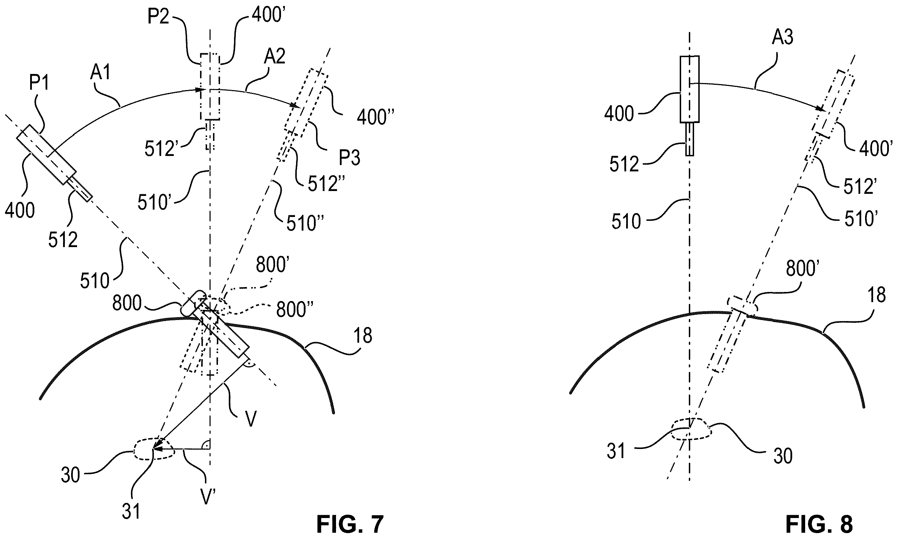

Figur 7 zeigt eine schematische Darstellung zur Ausrichtung der mit der Koppeleinheit 100 verbundenen Sterileinheit 400 zum Zieloperationsgebiet 30 nach einer ersten Vorgehensweise. Bei dieser Vorgehensweise wird der Manipulatorarm 16 zusammen mit der Koppeleinheit 100 in einem ersten Schritt derart ausgerichtet, dass die Längsachse 510 des Instrumentenschafts 512 durch die Instrumenteneinführöffnung des Trokars 800 verläuft. Bei dieser Ausrichtung ist der Betrag des Abstandsvektors V größer als ein voreingestellter Wert, sodass die Steuereinheit 46 eine Steuerinformation erzeugt, die angibt, dass die Längsachse 510 nicht durch das Zieloperationsgebiet 30 verläuft. Der Betrag des Abstandsvektors V ist jedoch so groß, dass er einen ersten voreingestellten Wert überschreitet, sodass weder ein optisches noch ein akustisches Signal ausgibt. Wird die Koppeleinheit 100 zusammen mit der Sterileinheit 400 bzw. zusammen mit der gesamten Instrumenteneinheit 300 von der Position P1 in Richtung des Pfeils A1 zur Position P2 verschwenkt, ist der Betrag des Abstandsvektors V' zwischen dem Mittelpunkt 31 des Zieloperationsgebiets 30 und der Längsachse 510' des Instrumentenschafts 512' geringer als ein erster voreingestellter Wert, sodass ein erstes akustisches Signal ausgibt und/oder Licht eines anderen Spektrums und/oder eines Teilspektrums des zuvor ausgestrahlten Lichts emittiert wird, sodass eine Bedienperson an der Farbänderung die Annäherung der Längsachse 510' an das Zieloperationsgebiet 30 einfach erfassen kann. Dann wird die Koppeleinheit 100 zusammen mit Sterileinheit 400' von der Position P2 weiter in Richtung des Pfeils A2 verschwenkt, bis der Instrumentenschaft 512" die Position P3 erreicht wird der Betrag des Abstandsvektors V' weiter verringert, bis er insbesondere den Wert Null erreicht hat, sodass ein zweiter voreingestellter Wert des Betrags des Abstandsvektors V' erreicht oder unterschritten wird. Ist das der Fall, wird ein zweites optisches und/oder akustisches Signal ausgegeben, durch das die Bedienperson über die korrekte Ausrichtung der Längsachse 510" in Bezug auf das Zieloperationsgebiet 30 informiert wird. Das zweite optische Signal kann gegenüber dem ersten optischen Signal unterschiedliches Wellenlängenspektrum oder eine unterschiedliche Wellenlänge aufweisen, sodass die Bedienperson durch die Farbänderung über die korrekte Ausrichtung des Manipulatorarms 16 informiert wird. Alternativ oder zusätzlich kann das zweite optische Signal eine gegenüber dem ersten optischen Signal verschiedene Blinkfrequenz haben.

Beim Vorsehen von zwei Grenzwerten, mit denen der Betrag des Abstandsvektors V jeweils verglichen wird, sind somit drei Zustände erfassbar, so dass einer Bedienperson bereits bei der Annäherung an das Zieloperationsgebiet 30 ein entsprechendes optisches und/oder akustisches Signal ausgegeben werden kann und einer korrekten Ausrichtung der Sterileinheit 400 auf das Zieloperationsgebiet 30 ein weiteres optisches und/oder akustisches Signal ausgegeben werden kann. Beim Vorsehen von nur einem Grenzwert können zwei Zustände einfach unterschieden werden, insbesondere das ein Abstand zwischen dem Zieloperationsgebiet 30 und der Längsachse 510 existiert, d.h. dass die Längsachse 510 nicht durch das Zieloperationsgebiet 30 verläuft, und dem Zustand, dass die Längsachse 510 durch das Zieloperationsgebiet 30 verläuft.

Figur 8 zeigt eine schematische Darstellung zur Ausrichtung der mit dem Manipulatorarm 16 verbundenen Sterileinheit 400 zum Zieloperationsgebiet 30 nach einer zweiten Vorgehensweise, bei der im Unterschied zur ersten Vorgehensweise die Längsachse 510 des Instrumentenschafts 512 in einem ersten Schritt derart ausgerichtet wird, dass sie durch das Zieloperationsgebiet 30 verläuft und der Bedienperson ein entsprechendes optisches und/oder akustisches Signal ausgegeben wird. Die Ausrichtung der Längsachse 510 zum Zieloperationsgebiet 30 kann dabei so erfolgen, wie beim Schritt 2 der ersten Vorgehensweise in Verbindung mit Figur 7 erläutert worden ist. Somit kann die Bedienperson auch hier optisch und/oder akustisch über den Abstand und/oder die Annährung der Längsachse 510 zum Zieloperationsgebiet 30 informiert werden. Verläuft die Längsachse 510 durch das Zieloperationsgebiet 30, wie dies für den Instrumentenschaft 512 in Figur 8 gezeigt ist, wird dieser dann in Richtung des Pfeils A3 verschwenkt, bis die Längsachse 510' des Instrumentenschafts 512' auf die Instrumentenöffnung des Trokars 800 trifft.

Anders als in Verbindung mit Figur 7 und 8 beschrieben, kann die Ausgabeeinheit 41, 47 und/oder die Anzeigeeinheit 44 auch nur ein optisches und/oder akustisches Signal ausgeben oder beispielsweise über eine Pulsfolge des akustischen Signals und/oder des optischen Signals einer Bedienperson über die Pulsweite und/oder die Pulsdauer eine Information zu geben, wie groß der Betrag des orthogonalen Abstandsvektors V zum Zieloperationsgebiet 30 und/oder zum Mittelpunkt 31 des Zieloperationsgebiets 30 ist.

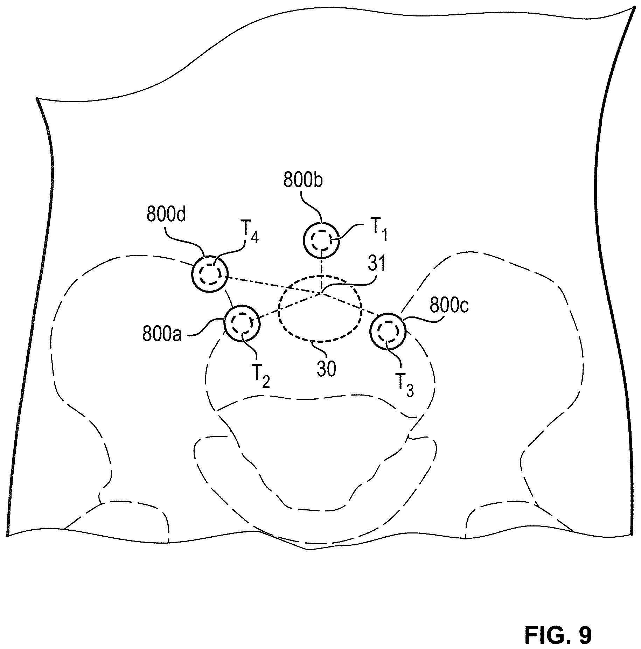

Figur 9 zeigt einen Ausschnitt des Körpers des Patienten 18 mit vier Körperöffnungen T1 bis T4, in die jeweils ein Trokar 800a bis 800d eingeführt ist. Durch den an der Eintrittsstelle T1 eingeführten Trokar 800b wird ein mit der Koppeleinheit 100b des Manipulatorarms 16b verbundenes Stabendoskop der Instrumenteneinheit 300b in den Körper des Patienten 18 eigeführt. Durch den an der Position T2 in den Körper des Patienten 18 eingeführten Trokar 800a wird ein chirurgisches Instrument 500a der mit der Koppeleinheit 100a des Manipulatorarms 16a verbundenen Instrumenteneinheit 300a in den Körper des Patienten 18 eingeführt. Durch einen an der Position T3 eingeführten Trokar 800c wird ein chirurgisches Instrument 500c der mit der Koppeleinheit 100c des Manipulatorarms 16c verbundenen Instrumenteneinheit 300c in den Körper des Patienten 18 eingeführt. Durch den an der Position T4 in den Körper des Patienten 18 eingeführten Trokar 800d wird ein chirurgisches Instrument 500d der mit der Koppeleinheit 100d des Manipulatorarms 16d verbundenen Instrumenteneinheit 300d in den Körper des Patienten 18 eingeführt.

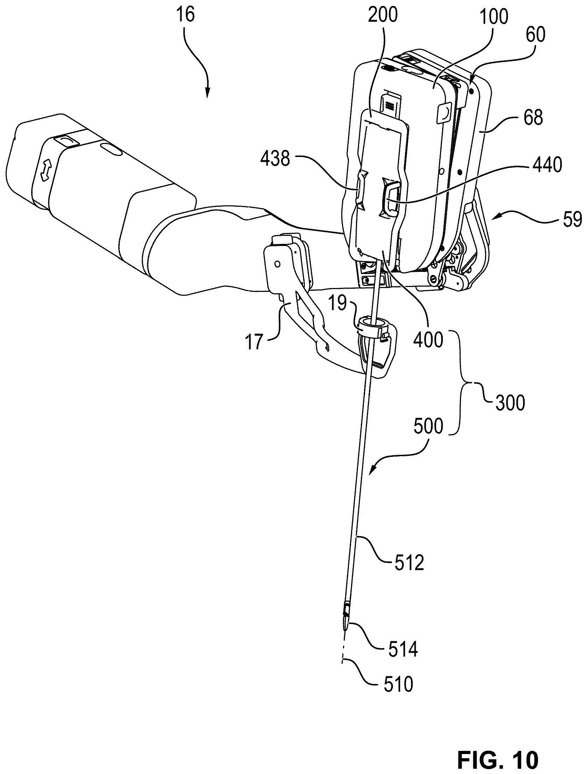

Figur 10 zeigt eine Anordnung mit dem proximalen Ende des Manipulatorarms 16 mit der Koppeleinheit 100 und der mit der Koppeleinheit 100 verbundenen Instrumenteneinheit 300 mit eingefahrener Teleskopanordnung 60 des Manipulatorarms16 gemäß einer zweiten Ausführungsform. Die Anordnung der zweiten Ausführungsform gemäß den Figuren 10 bis 12 unterscheidet sich von der in den Figuren 4 und 5 gezeigten und erläuterten ersten Ausführungsform lediglich dadurch, dass eine Trokar-Halterung 17 fest mit dem Manipulatorarm 16 verbunden ist. Die Trokar-Halterung 17 hat ein Verbindungselement 19 zum Verbinden der Trokar-Halterung 17 mit dem Trokar 800. Die Positionierung und Ausrichtung des Manipulatorarms 16 mit Hilfe der Instrumenteneinheit 300 erfolgt in gleicher Weise wie in Verbindung mit der ersten Ausführungsform beschrieben. Elemente mit gleicher Funktion oder gleichem Aufbau haben dieselben Bezugszeichen. In Figur 11 ist die Anordnung nach Figur 10 gezeigt, wobei die Trokar-Halterung 17 des Manipulatorarms 16 mit einem in den Patienten 18 eingeführten Trokar 800 verbunden ist und die Teleskopanordnung 60 einen ausgefahrenen Zustand hat. Der Instrumentenschaft 512 mit dem End-Effektor 514 ist ca. 2 cm tief in die Instrumentenöffnung des Trokars 800 eingeführt.

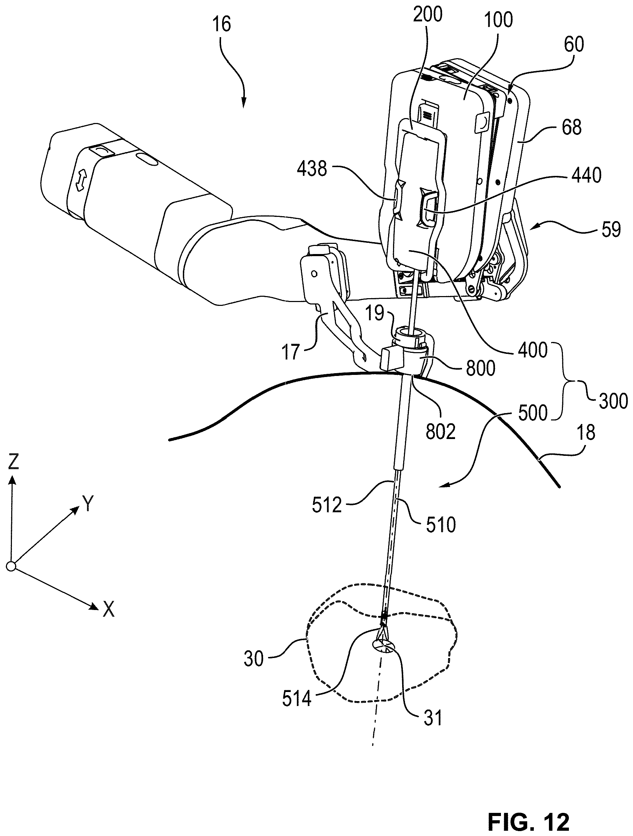

Figur 12 zeigt die Anordnung nach Figur 11, die Teleskopanordnung 60 in einem eingefahrenen Zustand dargestellt. Die Teleskopanordnung 60 ist von der in Figur 11 gezeigten ausgefahrenen Position in ihre in Figur 12 gezeigte eingefahrene Position bewegt worden, ohne dass die Ausrichtung des Manipulatorarms 16 und der Trokar-Halterung 17 geändert worden ist. Beim Einfahren und beim Ausfahren der Teleskopanordnung 60 erfolgt eine Ausgleichsbewegung durch ein Verschwenken der Koppeleinheit 100 gegenüber der Teleskopanordnung 60 und ein Verschwenken der Teleskopanordnung 60 zum Manipulatorarm 16 über das Koppelgetriebe 59, so dass das chirurgische Instrument 500 beim Ein- und beim Ausfahren der Teleskopanordnung 60 exakt entlang der Längsachse 510 des Instrumentenschafts 512 bewegt werden kann. Die Teleskopanordnung 60 kann so weit eingefahren werden, bis der Endeffektor 514 des chirurgischen Instruments 500 das Zieloperationsgebiet 30 erreicht oder in Richtung der Längsachse 510 des chirurgischen Instruments 500 über das Zieloperationsgebiet 30 hinausragt.

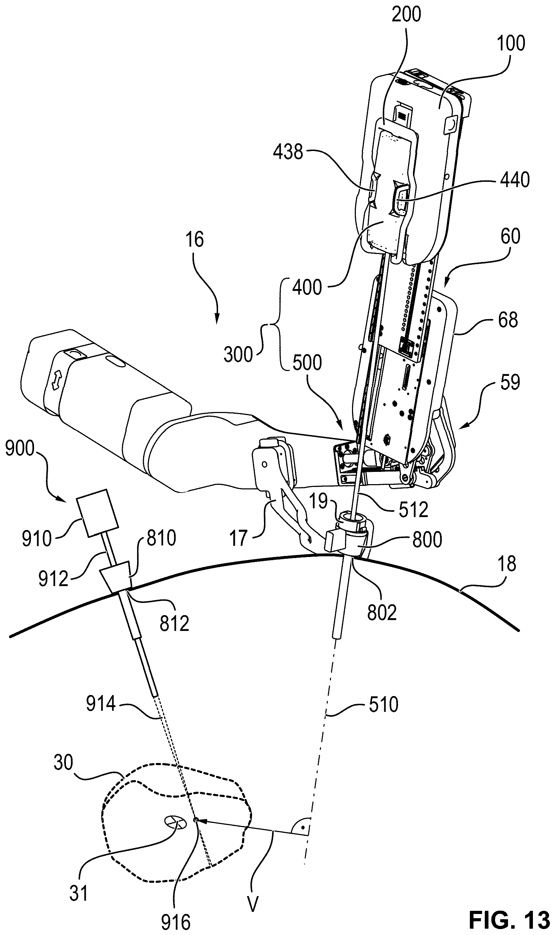

Figur 13 zeigt eine Anordnung mit einem Abschnitt eines Manipulatorarms 16 mit einer Koppeleinheit 100 und einem Endoskop 900 gemäß einer dritten Ausführungsform. Die Teleskopanordnung 60 des Manipulatorarms 16 ist in einem ausgefahrenen Zustand gezeigt. Eine Instrumenteneinheit 300 mit einem Chirurgischen Instrument 500 ist mit der Koppeleinheit 100 gekoppelt. Auch bei der dritten Ausführungsform ist eine Trokar-Halterung 17 vorgesehen, die einen in den Körper des Patienten 18 eingeführten Trokar 800 mit dem Manipulatorarm 16 verbindet. Auf die Trokar-Halterung 17 kann jedoch bei anderen Ausführungsformen auch verzichtet werden. Das Endoskop 900 ist ein Stabendoskop. Zumindest ein Teil der Abbildungsoptik des Stabendoskops 900 befindet sich in einem Stab 912, dessen proximales Ende über einen Trokar 810 über eine zweite Körperöffnung 812 in den Körper des Patienten 18 eingeführt ist. Mit Hilfe des Stabendoskops 900 werden Bilder zumindest eines Teils des Zieloperationsgebiets 30 aufgenommen. Das Stabendoskop 900 hat ein Kopfteil 910, über das das Stabendoskop 900 mit der Bedienkonsole 42 und/oder der Anzeigeeinheit 44 des Systems 10 zur robotergestützten Chirurgie verbunden ist. Dadurch kann einer Bedienperson, insbesondere einem Chirurgen, das mit Hilfe des Endoskops 900 aufgenommene Bild an der Bedienkonsole 42 bzw. der Anzeigeeinheit 44 angezeigt werden.

Über das Kopfteil 910 kann das Stabendoskop 900 auch mit einer weiteren Koppeleinheit 100 insbesondere eines weiteren Manipulatorarms 16 gekoppelt sein. Durch Strichlinien ist der Strahlengang 914 der Abbildungsoptik schematisch dargestellt. Die optische Achse der Abbildungsoptik des Endoskops 900, d. h. die optische Achse des Stabendoskops 900, liegt in der Mitte des Strahlengangs 914. Der Brennpunkt der Abbildungsoptik des Stabendoskops 900 ist mit 916 bezeichnet.

Im vorliegenden Ausführungsbeispiel wird der Abstandsvektor V zwischen der Längsachse 510 des Instrumentenschafts 512 orthogonale des chirurgischen Instruments 500 zum Brennpunkt 916 der Abbildungsoptik des Stabendoskops 900 ermittelt. Der Brennpunkt 916 dient somit als Zielpunkt. Der ermittelte orthogonale Abstandsvektor V gibt den aktuellen Abstand zwischen der Längsachse 510 des Instrumentenschafts 512 zum Brennpunkt 916 an. Die Auswertung der Position und die Ausrichtung des Manipulatorarms 16 mit dem chirurgischen Instrument 500 erfolgt dabei in gleicher Weise, wie dies in Verbindung mit den ersten beiden Ausführungsformen im Zusammenhang mit den Figuren 4 bis 12 bereits erläutert worden ist.

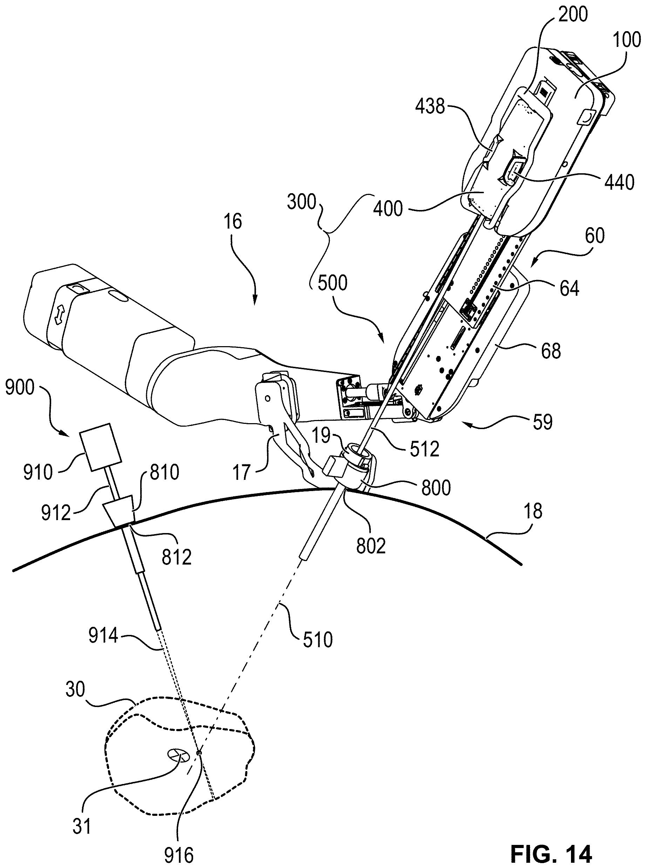

In Figur 13 ist das proximale Ende des Instrumentenschafts 512 mit dem Endeffektor 514 in die Instrumentenöffnung des Trokars 800 eingeführt worden. Vorzugsweise ragt der Endeffektor ca. 2 cm tief in die Instrumentenöffnung des Trokars 800 hinein. Bei anderen Ausführungsformen kann die Spitze des Endeffektors 514 auch bis zum proximalen Ende des Trokars 800 in dessen Instrumentenöffnung oder weniger als 2 cm in die Instrumentenöffnung des Trokars 800 eingeführt werden. Die Teleskopanordnung 60 befindet sich dabei in einem ausgefahrenen Zustand. Anschließend wird die Position der mit der Koppeleinheit 100 gekoppelten Instrumenteneinheit 300 zusammen mit zumindest einem Teil der Segmente des Manipulatorarms 16 so verändert, dass die Längsachse 510 des Instrumentenschafts 512 sich dem als Zielpunkt dienenden Brennpunkt 916 bis zu einem voreingestellten ersten und/oder zweiten Abstandswert angenähert hat, oder wie in Figur 14 gezeigt, die Längsachse 510 des Instrumentenschafts 512 durch den Brennpunkt 916 verläuft. Hierzu ist in Figur 14 dieTeleskopanordnung 60 des Manipulatorarms gegenüber der in Figur 13 gezeigten Position mit Hilfe des Koppelgetriebes 59 verschwenkt worden.

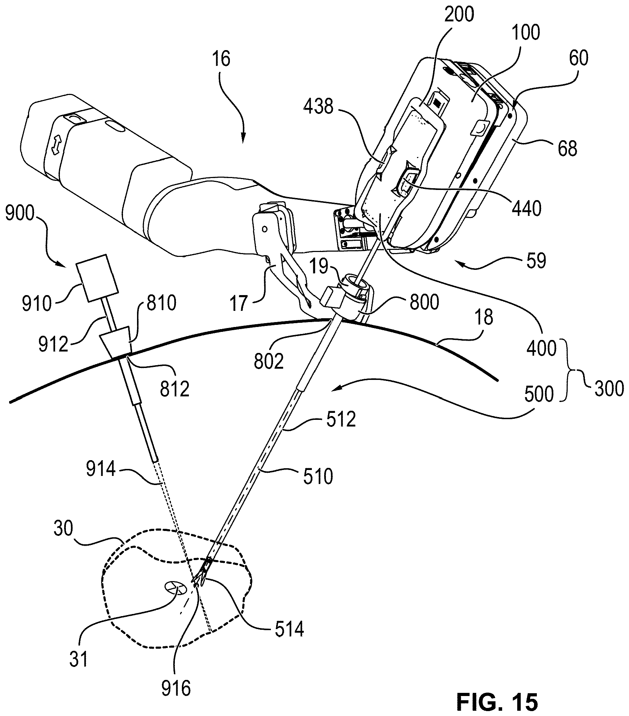

In Figur 15 ist die Anordnung nach den Figuren 13 und 14 mit eingefahrener Teleskopanordnung 60 gezeigt. Durch das Einfahren der Teleskopanordnung ist der Endeffektor 514 bis in den Sichtbereich des Stabendoskops 900 und in den Zieloperationsbereich 30 bewegt worden, und kann dort unter Sichtkontrolle betätigt und positioniert werden. Durch die beschriebene Vorgehensweise ist es somit möglich, die Position, d. h. die Ausrichtung und die Lage, eines einzusetzenden chirurgischen Instruments 500 bereits so voreinzustellen, dass es zum Zieloperationsgebiet 30 geführt wird und dort sicher in den Blickbereich (field of few) des Stabendoskops 900 gebracht wird. Der Zielpunkt oder das Zielgebiet können durch Koordinaten x_{Z}, y_{Z}, z_{Z} des Koordinatensystems X, Y, Z der Vorrichtung oder durch Koordinaten x'_{Z}, y'_{Z}, z'_{Z} des Patientenkoordinatensystems X', Y', Z' festgelegt sein und erforderlichenfalls in Koordinaten des jeweils anderen Koordinatensystems umgerechnet werden.

Auch bei der ersten und bei der zweiten Ausführungsform nach den Figuren 4 bis 12 kann anstatt des Zieloperationsgebiets 30 ein Zielpunkt, wie z.B. der Mittelpunkt 31 des festgelegten Zieloperationsgebiets 30 oder durch einen von der Lage des anderen chirurgischen Instruments 900 abhängiger Zielpunkt festgelegt sein. Auch kann anstatt des Endoskops 900 bei der dritten Ausführungsform ein anderes bildgebendes System zum Erfassen von Bildern zumindest eines Ausschnitts eines Zieloperationsgebiets 30 eingesetzt werden. Das Zielgebiet ist dann von der Lage des anderen bildgebenden Systems abhängig. Der Zielpunkt ist insbesondere ein Punkt im Schärfentiefebereich der Abbildungsoptik des Endoskops bzw. des bildgebenden Systems, wie beispielsweise der Brennpunkt der Abbildungsoptik oder ein Punkt zwischen dem Brennpunkt und dem proximalen Ende des Endoskops 900. Das Zielgebiet kann alternativ oder zusätzlich durch einen Abstand um einen Punkt festgelegt sein.

### Bezugszeichenliste

- 10: System
- 12: Manipulator
- 14: Stativ
- 16, 16a bis 16d: Manipulatorarm
- 17: Trokar-Halterung
- 18: Patient
- 19: Verbindungselement
- 20: Stativkopf
- 24: Stativfuß
- 28: Stativarm
- 30: Zieloperationsgebiet
- 31: Mittelpunkt des Zieloperationsgebiet
- 32: Operationstischsäule
- 34: Operationstisch
- 36: Steuereinheit des Operationstisches
- 38: Patientenlagerfläche
- 40: zentrale Steuereinheit der Vorrichtung
- 41: Ausgabeeinheit
- 42: Bedienkonsole
- 44: Anzeigeeinheit
- 46: Steuereinheit des Manipulators
- 47: Ausgabeeinheit
- 59: Koppelgetriebe
- 60: Teleskopanordnung
- 62, 64, 66: Abschnitte der Teleskopanordnung
- 68: Antriebseinheit
- 100, 100a bis 100d: Koppeleinheit
- 102: erste Übertragungsmittel
- 104: elektrisches Übertragungselement
- 106, 108: elektrischer Kontakt
- 109: optisches Übertragungselement
- 110: erstes translatorisches Antriebselement
- 112: zweites translatorisches Antriebselement
- 114: erstes rotatorisches Antriebselement
- 116: zweites rotatorisches Antriebselement
- 120: Koppelsensor
- 121: RFID-Lese- und Schreibeinheit
- 122, 124: Führungsnut
- 123, 125: vorderes Ende der Führungsnut
- 126: Rastnase
- 128: Rastelement
- 134: Entriegelungstaste
- 200: Sterilschleuse
- 201: Sterilfolie
- 202: Anschlussrand
- 204, 206: Führungsstift
- 208, 210: Schleusenklappe
- 300, 300a bis 300d: Instrumenteneinheit
- 400, 400a bis 400d, 400', 400": Sterileinheit
- 438, 440: Rast- und Betätigungselement
- 494: RFID-Transponder
- 500, 500a bis 500d: chirurgisches Instrument
- 510, 510', 510": Längsachse
- 512: Instrumentenschaft
- 514: Endeffektor
- 800: Trokar
- 802: Körperöffnung
- 810: Trokar
- 812: Körperöffnung
- 900: Stabendoskop
- 910: Kopfteil
- 912: Stab
- 914: Strahlengang
- 916: Brennpunkt
- A1 bis A3: Bewegungsrichtung
- P1 bis P3: Position
- T1 bis T4: geplante Körperöffnung
- V, V': Abstandsvektor
- x_{Z}, y_{Z}, z_{Z}: Koordinaten des Zielgebiets im Koordinatensystem der Vorrichtung
- X, Y, Z: Koordinatensystem der Vorrichtung
- X', Y', Z': Patientenkoordinatensystem
- x'_{Z}, y'_{Z}, z'_{Z}: Koordinaten des Zielgebiets im Patientenkoordinatensystem

## Patentansprüche

1. Vorrichtung zur robotergestützten Chirurgie,
mit einer Instrumenteneinheit (300), die ein chirurgisches Instrument (500) mit einem Instrumentenschaft (512) hat, dessen proximales, dem Patienten zugewandtes Ende durch eine Körperöffnung (802) eines Patienten (18) zu einem durch Koordinaten (x_{z}, y_{z}, z_{z}) eines Koordinatensystems (X, Y, Z) der Vorrichtung festgelegten Zielgebiet (30, 31, 916) führbar ist,
mit mindestens einer Koppeleinheit (100) eines Manipulatorarms (16), mit der wahlweise die Instrumenteneinheit (300) verbindbar ist,
mit einer Steuereinheit (40, 46) und mit einer Ausgabeeinheit (41, 47), **dadurch gekennzeichnet, dass**
- die Steuereinheit (40, 46) bei einer Verbindung der Instrumenteneinheit (300) mit der Koppeleinheit (100) den zur Längsachse (510) des Instrumentenschafts (512) des chirurgischen Instrumentes (500) orthogonalen Abstandsvektor (V) zwischen der Längsachse (510) und dem durch die Koordinaten (x_{z}, y_{z}, z_{z}) festgelegten Zielgebiet (30, 31, 916) ermittelt,
- die Steuereinheit (40, 46) eine erste Steuerinformation erzeugt, wenn der Betrag des ermittelten Abstandsvektors (V) einen ersten voreingestellten Wert hat und/oder unterschreitet, und
- dass die Ausgabeeinheit (41, 47) abhängig von der ersten Steuerinformation ein Signal ausgibt.

2. Vorrichtung nach Anspruch 1, **dadurch gekennzeichnet, dass** die Steuereinheit (40, 46) zumindest eine zweite Steuerinformation erzeugt, wenn der Betrag des ermittelten Abstandsvektors (V) einen zweiten voreingestellten Wert hat und/oder unterschreitet, und dass die Ausgabeeinheit (41, 47) ein Signal ausgibt.

3. Vorrichtung nach einem der vorhergehenden Ansprüche, **dadurch gekennzeichnet, dass** die Ausgabeeinheit (41, 47) der Vorrichtung auf Grund der ersten Steuerinformation ein erstes akustisches und/oder ein erstes optisches Signal ausgibt, und/oder dass die Ausgabeeinheit (41, 47) auf Grund der zweiten Steuerinformation nur ein zweites akustisches und/oder nur ein zweites optisches Signal ausgibt.

4. Vorrichtung nach Anspruch 3 oder 4, **dadurch gekennzeichnet, dass** das erste akustische Signal ein an- und abschwellender Ton oder eine Tonfolge mit einer ersten Wiederholfrequenz ist und dass das zweite akustische Signal ein Dauerton ist.

5. Vorrichtung nach einem der Ansprüche 3 oder 4, **dadurch gekennzeichnet, dass** das erste optische Signal ein blinkendes Lichtsignal mit einer ersten Blinkfrequenz ist und dass das zweite optische Signal ein Lichtsignal mit einer zweiten Blinkfrequenz ist, die auch null sein kann.

6. Verfahren zum Positionieren eines Manipulatorarms in einem Koordinatensystem (X, Y, Z) einer Vorrichtung zur robotergestützten Chirurgie, für einen geplanten chirurgischen Eingriff,
bei dem die Koordinaten (x_{z}, y_{z}, z_{z}) eines Zielgebietes (30, 31, 916) in einem Patienten (18) ermittelt werden,
bei dem eine Instrumenteneinheit (300) mit einer Koppeleinheit (100) des Manipulatorarms (16) verbunden wird,
wobei die Instrumenteneinheit (300) ein chirurgisches Instrument (500) umfasst, das einen Instrumentenschaft (512) mit einer Längsachse (510) hat,
bei dem bei einer Verbindung der Instrumenteneinheit (300) mit der Koppeleinheit (100) mit Hilfe einer Steuereinheit (40, 46) der zur Längsachse (510) orthogonale Abstandsvektor (V) zwischen der Längsachse (510) und dem durch die Koordinaten (x_{z}, y_{z}, z_{z}) festgelegte Zielgebiet (30, 31, 916) ermittelt wird, und
bei dem mit Hilfe einer Ausgabeeinheit (41, 47) ein erstes optisches und/oder akustisches Signal ausgegeben wird, wenn der Betrag des ermittelten Abstandsvektors (V) einen ersten voreingestellten Wert hat und/oder unterschreitet.

7. Verfahren nach Anspruch 6, **dadurch gekennzeichnet, dass** ein zweites optisches und/oder akustisches Signal ausgegeben wird, wenn der Betrag des ermittelten Abstandsvektors (V) einen zweiten voreingestellten Wert erreicht und/oder unterschreitet.

8. Verfahren nach Anspruch 6 oder 7, **dadurch gekennzeichnet, dass** der Manipulatorarm (16) so ausgerichtet wird, dass die Längsachse (514) des Instrumentenschafts (512) durch eine geplante operative Körperöffnung (802) eines Patienten (18) verläuft und das erste und/oder zweite optische und/oder akustische Signal ausgegeben wird.

9. Verfahren nach einem der vorhergehenden Ansprüche 6 bis 8, **dadurch gekennzeichnet, dass** der Manipulatorarm (16) in einem ersten Schritt so ausgerichtet wird, dass die Längsachse (510) des Instrumentenschafts (512) durch eine geplante operative Körperöffnung (802) des Patienten (18) verläuft, und dass der Manipulatorarm (16) in einem zweiten Schritt so bewegt wird, bis der mit Hilfe der Steuereinheit (40, 46) ermittelte Betrag des Abstandsvektors (V) zwischen der durch die geplante operative Körperöffnung (802) des Patienten (18) verlaufende Längsachse (510) und dem durch die Koordinaten (x_{z}, y_{z}, z_{z}) festgelegten Zielgebiet (30, 31, 916) den ersten und/oder zweiten Wert erreicht oder unterschreitet,
wobei der Manipulatorarm (16) automatisch durch die Vorrichtung selbst und/oder manuell bewegt wird.

10. Verfahren nach einem der vorhergehenden Ansprüche 6 bis 9, **dadurch gekennzeichnet, dass** der Manipulatorarm (16) in einem ersten Schritt so ausgerichtet wird, dass der mit Hilfe der Steuereinheit (40, 46) ermittelte Betrag des Abstandsvektors (V) zwischen der Längsachse (510) und dem durch die Koordinaten (x_{z}, y_{z}, z_{z}) festgelegten Zielgebiet (30, 31, 916) den ersten und/oder zweiten Wert erreicht oder unterschreitet,
und dass der Manipulatorarm (16) in einem zweiten Schritt so ausgerichtet wird, dass die Längsachse (510) des Instrumentenschafts (512) durch die geplante operative Körperöffnung (802) des Patienten (18) verläuft,
wobei der Manipulatorarm (16) automatisch durch die Vorrichtung selbst und/oder manuell bewegt wird.

11. Vorrichtung oder Verfahren nach einem der vorhergehenden Ansprüche, **dadurch gekennzeichnet, dass** das Zielgebiet durch ein Zieloperationsgebiet (30), durch einen Mittelpunkt (31) eines Zieloperationsgebietes (30) oder durch die Lage eines anderen chirurgischen Instruments (300, 900) festgelegt ist.

12. Vorrichtung oder Verfahren nach einem der vorhergehenden Ansprüche, **dadurch gekennzeichnet, dass** das Zielgebiet durch die Lage eines zumindest teilweise in den Körper des Patienten (18) eingeführten Endoskops (900) oder eines anderen bildgebenden Systems zum Erfassen von Bildern zumindest eines Ausschnitts eines Zieloperationsgebiets (30) festgelegt ist, vorzugsweise durch die optische Achse und/oder den Brennpunkt (916) der Abbildungsoptik des Endoskops (900) bzw. des bildgebenden Systems.

## Claims

1. An apparatus for robot-assisted surgery, comprising
an instrument unit (300) having a surgical instrument (500) with an instrument shaft (512), the proximal end of which facing the patient is passable through a body orifice (802) of a patient (18) to a target area (30, 31, 916) defined by coordinates (x_{z}, y_{z}, z_{z}) of a coordinate system (X, Y, Z) of the apparatus,
at least one coupling unit (100) of a manipulator arm (16), to which optionally the instrument unit (300) is connectable,
a control unit (40, 46) and an output unit (41, 47), **characterized in that**
- the control unit (40, 46), when the instrument unit (300) is connected to the coupling unit (100), determines the distance vector (V), which is orthogonal to the longitudinal axis (510) of the instrument shaft (512) of the surgical instrument (500), between the longitudinal axis (510) and the target area (30, 31, 916) defined by the coordinates (x_{z}, y_{z}, z_{z}),
- the control unit (40, 46) generates a first control information when the amount of the determined distance vector (V) has and/or falls below a first preset value, and
- the output unit (41, 47) outputs a signal dependent on the first control information.

2. The apparatus according to claim 1, **characterized in that** the control unit (40, 46) generates at least a second control information, when the amount of the determined distance vector (V) has and/or falls below a second preset value, and that the output unit (41, 47) outputs a signal.

3. The apparatus according to one of the preceding claims, **characterized in that** the output unit (41, 47) of the apparatus outputs a first acoustic and/or a first optical signal on the basis of the first control information, and/or that the output unit (41, 47) outputs only a second acoustic and/or only a second optical signal on the basis of the second control information.

4. The apparatus according to claim 3 , **characterized in that** the first acoustic signal is a swelling and falling tone or a tone sequence with a first repetition rate and that the second acoustic signal is a continuous tone.

5. The apparatus according to one of the claims 3 or 4, **characterized in that** the first optical signal is a blinking light signal with a first blinking rate and that the second optical signal is a light signal with a second blinking rate, which may also be zero.

6. A method for positioning a manipulator arm in a coordinate system (X, Y, Z) of an apparatus for robot-assisted surgery, for a planned surgical intervention,
in which the coordinates (x_{z}, y_{z}, z_{z}) of a target area (30, 31, 916) in a patient (18) are determined,
in which an instrument unit (300) is connected to a coupling unit (100) of the manipulator arm (16),
wherein the instrument unit (300) comprises a surgical instrument (500) having an instrument shaft (512) with a longitudinal axis (510),
in which, when the instrument unit (300) is connected to the coupling unit (100), the distance vector (V), which is orthogonal to the longitudinal axis (510), between the longitudinal axis (510) and the target area (30, 31, 916) defined by the coordinates (x_{z}, y_{z}, z_{z}) is determined by means of a control unit (40, 46), and
in which a first optical and/or acoustic signal is output by means of an output unit (41, 47) when the amount of the determined distance vector (V) has and/or falls below a first preset value.

7. The method according to claim 6, **characterized in that** a second optical and/or acoustic signal is output when the amount of the determined distance vector (V) has reached and/or falls below a second preset value.

8. The method according to claim 6 or 7, **characterized in that** the manipulator arm (16) is oriented such that the longitudinal axis (510) of the instrument shaft (512) runs through a planned operative body orifice (802) of a patient (18) and the first and/or second optical and/or acoustic signal is output.

9. The method according to one of the preceding claims 6 to 8, **characterized in that** the manipulator arm (16) is oriented in a first step such that the longitudinal axis (510) of the instrument shaft (512) runs through a planned operative body orifice (802) of the patient (18), and that the manipulator arm (16) is moved in a second step until the amount of the distance vector (V), determined by means of the control unit (40, 46), between the longitudinal axis (510) running through the planned operative body orifice (802) of the patient (18) and the target area (30, 31, 916) defined by the coordinates (x_{z}, y_{z}, z_{z}) reaches or falls below the first and/or second value,
wherein the manipulator arm (16) is moved automatically by the apparatus itself and/or manually.

10. The method according to one of the preceding claims 6 to 9, **characterized in that** the manipulator arm (16) is oriented in a first step such that the amount of the distance vector (V), which is determined by means of the control unit (40, 46), between the longitudinal axis (510) and the target area (30, 31, 916) defined by the coordinates (x_{z}, y_{z}, z_{z}) reaches or falls below the first and/or second value,
and that the manipulator arm (16) is oriented in a second step such that the longitudinal axis (510) of the instrument shaft (512) runs through the planned operative body orifice (802) of the patient (18),
wherein the manipulator arm (16) is moved automatically by the apparatus itself and/or manually.

11. The apparatus or the method according to one of the preceding claims, **characterized in that** the target area is defined by a target surgical area (30), by a center (31) of a target surgical area (30) or by the position of another surgical instrument (300, 900).

12. The apparatus or method according to one of the preceding claims, **characterized in that** the target area is defined by the position of an endoscope (900) at least partly inserted into the body of the patient (18) or another imaging system for capturing images of at least a detail of a target surgical area (30), preferably by the optical axis and/or the focal point (916) of the imaging optics of the endoscope (900) or the imaging system.

## Revendications

1. Dispositif de chirurgie robotique,
comprenant une unité d'instruments (300) qui présente un instrument chirurgical (500) pourvu d'une tige d'instrument (512) dont l'extrémité proximale tournée vers le patient peut être guidée à travers un orifice corporel (802) d'un patient (18) en direction d'une zone cible (30, 31, 916) établie par les coordonnées (x_{z}, y_{z}, z_{z}) d'un système de coordonnées (X, Y, Z) du dispositif,
comprenant au moins une unité d'accouplement (100) d'un bras manipulateur (16), à laquelle l'unité d'instruments (300) peut être reliée sélectivement,
comprenant une unité de commande (40, 46) et une unité de sortie (41, 47),
**caractérisé en ce que**
- l'unité de commande (40, 46) détermine, lorsque l'unité d'instruments (300) est reliée à l'unité d'accouplement (100), le vecteur de distance (V) perpendiculaire à l'axe longitudinal (510) de la tige d'instrument (512) de l'instrument chirurgical (500) entre l'axe longitudinal (510) et la zone cible (30, 31, 916) établie par les coordonnées (x_{z}, y_{z}, z_{z}),
- l'unité de commande (40, 46) génère une première information de commande, lorsque la grandeur du vecteur de distance (V) déterminé est inférieure ou égale à une première valeur prédéfinie, et
- **en ce que** l'unité de sortie (41, 47) émet un signal en fonction de la première information de commande.

2. Dispositif selon la revendication 1, **caractérisé en ce que** l'unité de commande (40, 46) génère au moins une deuxième information de commande, lorsque la grandeur du vecteur de distance (V) déterminé est inférieure ou égale à une deuxième valeur prédéfinie, et **en ce que** l'unité de sortie (41, 47) émet un signal.

3. Dispositif selon l'une quelconque des revendications précédentes, **caractérisé en ce que** l'unité de sortie (41, 47) du dispositif émet un premier signal acoustique et/ou un premier signal optique sur la base de la première information de commande, et/ou **en ce que** l'unité de sortie (41, 47) émet uniquement un deuxième signal acoustique et/ou uniquement un deuxième signal optique sur la base de la deuxième information de commande.

4. Dispositif selon la revendication 3 ou 4, **caractérisé en ce que** le premier signal acoustique est un son qui va en montant et qui va en descendant ou une succession de sons présentant une première fréquence de répétition et **en ce que** le deuxième signal acoustique est un son continu.

5. Dispositif selon l'une quelconque des revendications 3 ou 4, **caractérisé en ce que** le premier signal optique est un signal lumineux clignotant présentant une première fréquence de clignotement et **en ce que** le deuxième signal optique est un signal lumineux présentant une deuxième fréquence de clignotement qui peut également être nulle.

6. Procédé pour le positionnement d'un bras manipulateur dans un système de coordonnées (X, Y, Z) d'un dispositif de chirurgie robotique, pour une intervention chirurgicale planifiée,
selon lequel les coordonnées (x_{z}, y_{z}, z_{z}) d'une zone cible (30, 31, 916) dans un patient (18) sont déterminées,
selon lequel une unité d'instruments (300) est reliée à une unité d'accouplement (100) du bras manipulateur (16),
dans lequel l'unité d'instruments (300) comporte un instrument chirurgical (500) qui présente une tige d'instrument (512) pourvue d'un axe longitudinal (510),
selon lequel, lorsque l'unité d'instruments (300) est reliée à l'unité d'accouplement (100), le vecteur de distance (V) perpendiculaire à l'axe longitudinal (510) entre l'axe longitudinal (510) et la zone cible (30, 31, 916) établie par les coordonnées (x_{z}, y_{z}, z_{z}) est déterminé à l'aide d'une unité de commande (40, 46), et
selon lequel un premier signal optique et/ou acoustique est émis à l'aide d'une unité de sortie (41, 47), lorsque la grandeur du premier vecteur de distance (V) déterminé est inférieure et/ou égale à une première valeur prédéfinie.

7. Procédé selon la revendication 6, **caractérisé en ce qu'**un deuxième signal optique et/ou acoustique est émis lorsque la grandeur du vecteur de distance (V) déterminé est inférieure et/ou égale à une deuxième valeur prédéfinie.

8. Procédé selon la revendication 6 ou 7, **caractérisé en ce que** le bras manipulateur (16) est orienté de telle sorte que l'axe longitudinal (514) de la tige d'instrument (512) s'étend à travers un orifice corporel (802) d'un patient (18) dont l'opération a été planifiée et que le premier et/ou deuxième signal optique et/ou acoustique est émis.

9. Procédé selon l'une quelconque des revendications précédentes 6 à 8, **caractérisé en ce que** le bras manipulateur (16) est orienté au cours d'une première étape de telle sorte que l'axe longitudinal (510) de la tige d'instrument (512) s'étend à travers un orifice corporel (802) du patient (18) dont l'opération a été planifiée, et **en ce que** le bras manipulateur (16) est déplacé au cours d'une deuxième étape jusqu'à ce que la valeur du vecteur de distance (V) entre l'axe longitudinal (510) s'étendant à travers l'orifice corporel (802) du patient (18) dont l'opération a été planifiée et la zone cible (30, 31, 916) établie par les coordonnées (x_{z}, y_{z}, z_{z}), laquelle valeur a été déterminée à l'aide de l'unité de commande (40, 46), soit inférieure ou égale à la première et/ou deuxième valeur,
dans lequel le bras manipulateur (16) est déplacé automatiquement par le dispositif lui-même et/ou manuellement.

10. Procédé selon l'une quelconque des revendications précédentes 6 à 9, **caractérisé en ce que** le bras manipulateur (16) est orienté au cours d'une première étape de telle sorte que la valeur du vecteur de distance (V) entre l'axe longitudinal (510) et la zone cible (30, 31, 916) établie par les coordonnées (x_{z}, y_{z}, z_{z}), laquelle valeur a été déterminée à l'aide de l'unité de commande (40, 46), est inférieure ou égale à la première et/ou deuxième valeur,
et **en ce que** le bras manipulateur (16) est orienté au cours d'une deuxième étape de telle sorte que l'axe longitudinal (510) de la tige d'instrument (512) s'étend à travers l'orifice corporel (802) du patient (18) dont l'opération a été planifiée,
dans lequel le bras manipulateur (16) est déplacé automatiquement par le dispositif lui-même et/ou manuellement.

11. Dispositif ou procédé selon l'une quelconque des revendications précédentes, **caractérisé en ce que** la zone cible est établie par une zone opératoire cible (30), par un centre (31) d'une zone opératoire cible (30) ou par la position d'un autre instrument chirurgical (300, 900).

12. Dispositif ou procédé selon l'une quelconque des revendications précédentes, **caractérisé en ce que** la zone cible est établie par la position d'un endoscope (900) introduit au moins en partie dans le corps du patient (18) ou d'un autre système d'imagerie destiné à acquérir des images d'au moins une partie d'une zone opératoire cible (30), de préférence à travers l'axe optique et/ou le foyer (916) de l'optique de reproduction de l'endoscope (900) ou du système d'imagerie.
